(19) Europäisches Patentamt
European Patent Office
Office européen des brevets

(11) Veröffentlichungsnummer: **0 201 896**
**B1**

(12) # EUROPÄISCHE PATENTSCHRIFT

(45) Veröffentlichungstag der Patentschrift: **02.05.90**

(51) Int. Cl.$^5$: **C 09 B 29/033,** D 06 P 1/18

(21) Anmeldenummer: **86106413.7**

(22) Anmeldetag: **12.05.86**

(54) **Farbstoffe mit Thiophenresten.**

(30) Priorität: **14.05.85 DE 3517365**
**20.09.85 DE 3533546**
**02.10.85 DE 3535133**

(43) Veröffentlichungstag der Anmeldung:
**20.11.86 Patentblatt 86/47**

(45) Bekanntmachung des Hinweises auf die Patenterteilung:
**02.05.90 Patentblatt 90/18**

(84) Benannte Vertragsstaaten:
**AT BE CH DE FR GB IT LI NL SE**

(56) Entgegenhaltungen:
**FR-A-2 184 828**
**FR-A-2 190 883**
**FR-A-2 313 431**
**GB-A-1 436 176**
**GB-A-1 578 733**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft**
**Carl-Bosch-Strasse 38**
**D-6700 Ludwigshafen (DE)**

(72) Erfinder: **Hansen, Günter, Dr.**
**Alwin-Mittasch-Platz 8**
**D-6700 Ludwigshafen (DE)**
Erfinder: **Schefczik, Ernst, Dr.**
**Dubliner Strasse 7**
**D-6700 Ludwigshafen (DE)**
Erfinder: **Etzbach, Karl-Heinz, Dr.**
**Bensheimer Ring 9 a**
**D-6710 Frankenthal (DE)**
Erfinder: **Reichelt, Helmut, Dr.**
**Weinbachstrasse 20**
**D-6701 Niederkirchen (DE)**
Erfinder: **Loeffler, Hermann**
**Haydnstrasse 23**
**D-6720 Speyer (DE)**

(56) References cited:
**CHEMICAL ABSTRACTS, Band 100, Nr. 26, Juni 1984, Seite 87, Zusammenfassung Nr. 211664r, Columbus, Ohio, US; & JP - A - 59 42 376 (NIPPON KAYAKU CO., LTD.) 08-03-1984**

**EP 0 201 896 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(56) References cited:

CHEMICAL ABSTRACTS, Band 102, Nr. 16, April 1985, Seite 78, Zusammenfassung Nr. 133543a, Columbus, Ohio, US; & JP - A - 59 204 658 (GOSEI SENRYO GIJUTSU KENKYU KUMIAI) 20-11-1984

Research Disclosure, October (1980), 19826
M.H. ELNAGDI et al. J. Heterocyclic Chem. 16, 1541 (1979)

K. GEWALD, G. NEUMANN, Chem. Ber. 101, 1933 (1968)

K. GEWALD, Chem. Ber. 98, 3571 (1965)

J.L-ISIDOR et al J.Org. Chem. 38, 3615 (1973)

D.E. WOLF und K. FOLKERS "The preparation of Thiophenes and Tetrahydrothiophenes" Org. Recict. 6, 410-468 (1951)

G.WAGNER, D. SINGER Z.Chem. 2, 305 (1962)
K. GEWALD Z. Chem. 7, 186 (1967)

"The Chemistry of Heterocyclic Compounds - Thiophene and its Derivatives"; Interscience Publ. (1952)

**Beschreibung**

Die Erfindung betrifft Verbindungen der allgemeinen Formel I

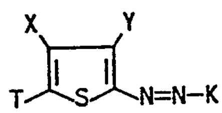

in der

X Fluor, Chlor, Brom, $SO_2E$, OH, $OCH_3$, $OC_2H_5$, $OC_3H_7$, $OC_4H_9$, $OCH_2C_6H_5$, $OC_6H_{11}$, $OC_6H_5$, $OC_6H_4CH_3$, $OC_6H_4Cl$, SH, $SCH_3$, $SC_2H_5$, $SC_3H_7$, $SC_4H_9$, $SCH_2C_6H_5$, $SC_2H_4OH$, $SCH_2COOCH_3$, $SCH_2COOC_2H_5$, $SC_6H_{11}$, $SC_6H_5$ oder $SC_6H_4CH_3$;

E Alkyl, Alkenyl, Cycloalkyl, Aralkyl, Aryl, Chlor, OH, $CH_3O$, $C_2H_5O$, $C_3H_7O$, $C_4H_9O$, $C_6H_5—CH_2O$, $C_6H_5—CH_2—CH_2O$, $C_6H_5O$, $ClC_6H_4O$, $CH_3C_6H_4O$, $NH_2$, $NHCH_3$, $N(CH_3)_2$, $NHC_2H_5$, $N(C_2H_5)_2$, $NHC_4H_9$, $N(C_4H_9)_2$ $NHC_6H_5$, $NHC_6H_4—CH_3$, $NHC_6H_4Cl$ oder $NCH_3C_6H_5$,

Y Cyan, Nitro, Alkanyoyl Aroyl, Alkylsulfonyl, Arylsulfonyl, Carboxyl, Carbonester oder gegebenenfalls substituiertes Carbamoyl,

T Wasserstoff, $C_1$— bis $C_4$-Alkyl, NO, $NO_2$, $SO_3H$, CHO, CN, $CH_3CO$, $C_2H_5CO$, $C_6H_5CO$, $CH_3SO_2$, $C_2H_5SO_2$, $C_6H_5SO_2$ oder ein Rest der Formel —CH=B, wobei B der Rest einer methylenaktiven Verbindung oder eines Amins ist, und

K den Rest einer Kupplungskomponente bedeutet.

Reste E sind beispeilsweise $CH_3$, $C_2H_5$, $C_3H_7$, $C_4H_9$, $C_6H_{13}$, $C_6H_{11}$, $C_8H_{17}$, $C_6H_5—CH_2$, $C_6H_5—CH_2—CH_2$, $C_6H_5$, $Cl—C_6H_4$ oder $C_4H_9—C_6H_4$.

Reste Y sind im einzelnen neben den bereits genannten z.B.:
$CH_3CO$, $C_2H_5CO$, $C_3H_7CO$, $C_4H_9CO$, $C_5H_{11}CO$, $C_7H_{15}CO$,

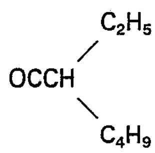

,

$C_6H_5CO$, $CH_3C_6H_4CO$, $ClC_6H_4CO$, $(CH_3)_2C_6H_3CO$, $H_3COC_6H_4CO$, $Cl_2C_6H_3CO$, $CH_3SO_2$, $C_2H_5SO_2$, $C_4H_9SO_2$. $C_6H_5SO_2$, $CH_3C_6H_4SO_2$, $ClC_6H_4SO_2$, $COOCH_3$, $COOC_2H_5$, $COOC_3H_7$, $COOC_4H_9$, $COOC_6H_{13}$, $COOC_8H_{17}$,

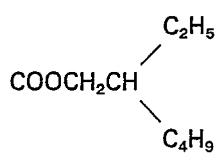

$COOC_2H_4OH$, $COOC_3H_6OH$, $COOC_2H_4OCH_3$, $COOC_2H_4OC_2H_5$, $COOC_2H_4OC_4H_9$, $COOC_6H_5$, $COOC_6H_4CH_3$, $CONH_2$, $CONHCH_3$, $CONHC_2H_5$, $CONHC_4H_9$, $CONHC_6H_{13}$, $CONHC_8H_{17}$, $CON(CH_3)_2$, $CON(C_2H_5)_2$, $CON(C_3H_7)_2$,

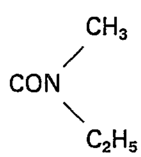

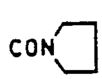 , 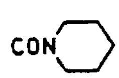 oder 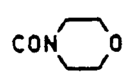 .

Methylenaktive Verbindungen der Formel $H_2B$ sind beispielsweise Verbindungen der Formel

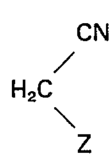

wobei Z Cyan, Nitro, Alkanoyl, Aroyl, Alkylsulfonyl, Arylsulfonyl, Carboxyl, Carbonester oder gegebenenfalls, substituiertes Carbamoyl ist oder einen Rest der Verbindungen der Formeln

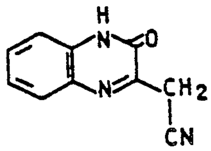, 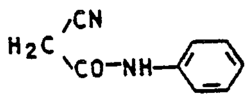, 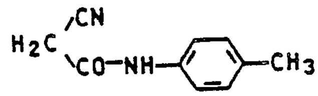, 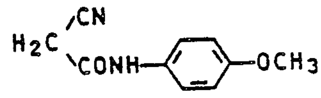,

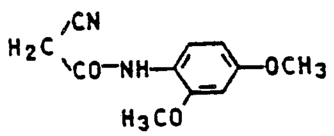, 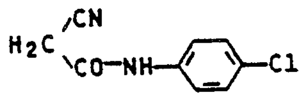, 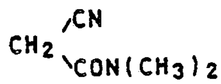, 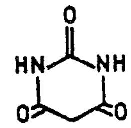,

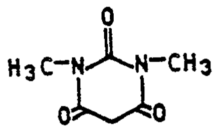, 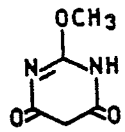, 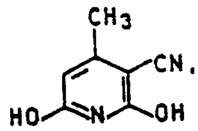, 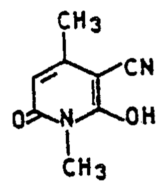, 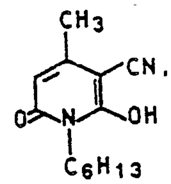,

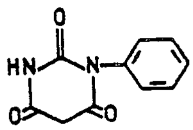, 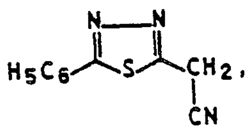, 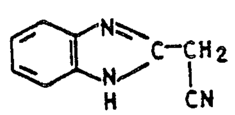, 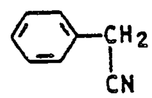, 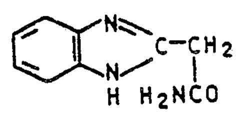

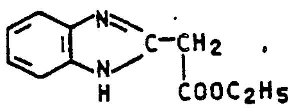, 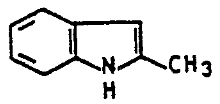, 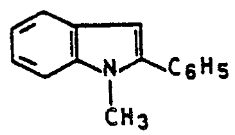, 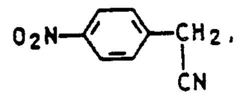,

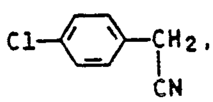, 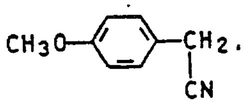, 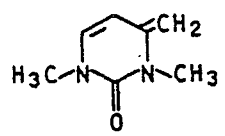, $CH_3-NO_2$, $C_2H_5-NO_2$,

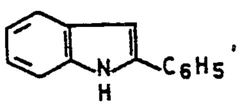, 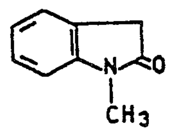, 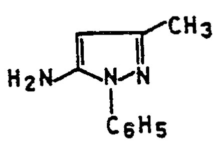, 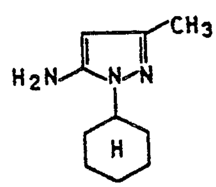,

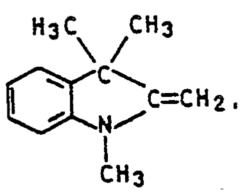, 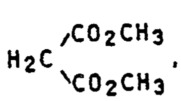, 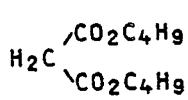 oder 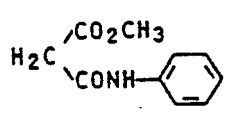

bedeutet.

Einzelne wichtige Verbindungen der Formel

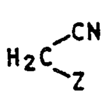 sind z.B.:

$H_2C(CN)_2$, 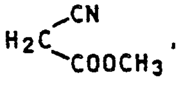, 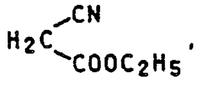, 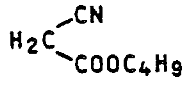, 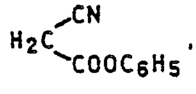,

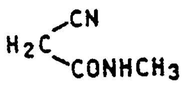, 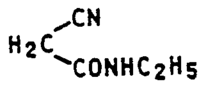, 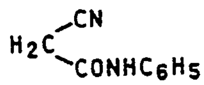, 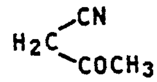 oder 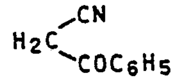

sowie 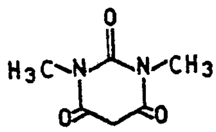, 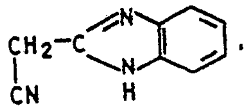. 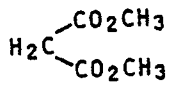, 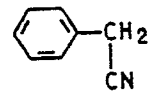

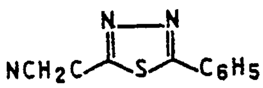 oder 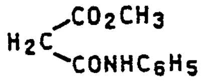.

Aminreste B sind z.B. $=N—C_6H_5$ $=N—C_6H_4CH_3$ oder allgemein Reste der Schiff schen Basen der Amine.

Als Alkylreste für T sind z.B. $CH_3$, $C_2H_5$, $C_3H_7$ oder $C_4H_9$ zu nennen.

Zur Herstellung der Diazokomponenten mit T = H oder $C_1$- bis $C_4$-Alkyl kann man Verbindungen der Formel II

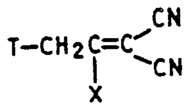

mit Schwefel abgebenden Verbindungen umsetzen. In die Diazokomponenten mit T = H können durch elektrophile Substitution Reste T nach den üblichen Methoden eingeführt werden.

Weiterhin kann die Diazokomponente mit T = H und X = OH auch dadurch hergestellt werden, daß man die Verbindung der Formel

$$ClCH_2COCl$$

mit Malodinitril umsetzt und anschließend mit einem Sulfid reagieren läßt.

Die Herstellung der Diazokomponenten ist im einzelnen in der EP—A—0193885 beschrieben.

Die Kupplingskomponenten der Formel HK stammen vorzugsweise aus der Anilin-, α-Naphthylamin-, Pyrazol-, Aminopyrazol-, Indol-, Thiazol-, Thiophen-, Phenol-, Naphthol-, Tetrahydrochinolin, Pyridon- oder Pyridinreihe, wobei solche der Anilin-, Pyrazol-, Thiophen- oder Thiazolreihe bevorzugt sind.

Die Kupplungskomponenten HK entsprechen insbesondere den allgemeinen Formeln

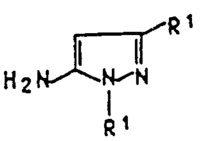, 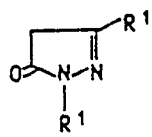, 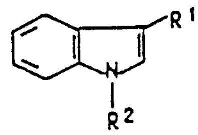, 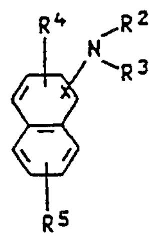, 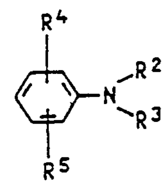,

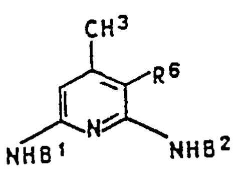, 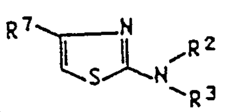, 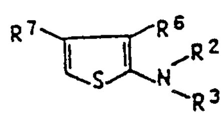 oder 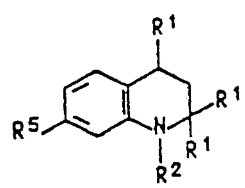,

wobei

$B^1$ Wasserstoff oder $B^2$

$B^2$ gegebenenfalls substituiertes Alkyl, Cycloakyl, Alkenyl, Aryl oder Acyl,

$R^1$ Wasserstoff, Alkyl, Aralkyl oder Aryl

$R^2$ Wasserstoff oder $R^3$,

$R^3$ gegebenenfalls substituiertes Alkyl, Cycloakyl, Alkenyl, Aralkyl oder Aryl,

$R^4$ und $R^5$ unabhangig voneinander Wasserstoff, Alkyl, Alkoxy, Phenoxy, Halogen, Alkylsulfonylamino, Dialkylaminosulfonylamino oder Acylamino,

$R^6$ Cyan, Carbamoyl, Nitro, Acetyl oder Carbalkoxy und

$R^7$ gegebenenfalls substituiertes Phenyl, Alkyl, Aralkyl, Halogen, $C_1$- bis $C_{10}$-Alkoxy, Phenoxy, Benzyloxy, $C_1$- bis $C_4$-Alkoxy-ethoxy, $C_1$- bis $C_4$-Alkyl- oder Phenylmercapto sind.

Reste $B^2$ sind neben den bereits genannten z.B. $C_1$- bis $C_6$-Alkylgruppen, die durch Chlor, Brom, Hydroxy, $C_1$- bis $C_8$-Alkoxy, Phenoxy, Phenyl, Cyan, Carboxy.

$C_1$- bis $C_8$-Alkanoyloxy, $C_1$- bis $C_8$-Alkoxy-$C_1$—$C_4$-alkoxy, Benzoyloxy, o-, m-, p-Methylbenzoyloxy, o-, m-, p-Chlorbenzoyloxy, $C_1$- bis $C_8$-Alkoxyalkanoyloxy, Phenoxyalkanoyloxy; $C_1$- bis $C_8$-Alkoxycarbonyloxy, $C_1$- bis $C_8$-Alkoxyalkoxycarbonyloxy, Benzyloxycarbonyloxy, Phenethyloxycarbonyloxy, Phenoxyethoxycarbonyloxy, $C_1$- bis $C_8$-Alkylaminocarbonyloxy, Cyclohexylaminocarbonyloxy, Phenylaminocarbonyloxy, $C_1$- bis $C_8$-Alkoxycarbonyl, $C_1$- bis $C_8$-Alkoxyalkoxycarbonyl, Phenoxycarbonyl, Benzyloxycarbonyl, Phenoxy-$C_1$—$C_4$-alkoxy oder Phenethyloxycarbonyl substituiert sein können sowie Phenyl oder Cyclohexyl.

Reste $B^2$ sind z.B. im einzelnen:

1. Gegebenenfalls substituierte Alkylreste wie:

```
CH3, C2H5, C3H7, CH(CH3)2, C4H9, CH2CH(CH3)2, CH-C2H5, C5H11,
                                              |
                         CH3                  CH3                CH3
                         |                                       |
CH-CH(CH3)2, CH(C2H5)2, CH-C3H7, CH——CH-CH3, C2H4-CH(CH3)2, CH2-C-CH3,
|                                 |   |                          |
CH3                               CH3 CH3                        CH3

                           ╱CH(CH3)2
C6H13, C7H15, CH-C4H9, CH           , CH-C2H4-CH(CH3)2, C8H17,
              |            ╲CH(CH3)2  |
              C2H5                    CH3

CH2-CH-C4H9 und CH-C3H6-CH(CH3)2;
    |           |
    C2H5        CH3                       CH3           CH3
                                          |             |
C2H4OH, C3H6OH, CH2-CH-OH, CH-CH2-OH, C4H8OH, CH-C2H4-OH, CH-CH2-OH,
                    |      |
                    CH3    CH3

C6H12OH, CH-C3H6C(CH3)2;
         |      |
         CH3    OH

C2H4OCH3, C2H4OC2H5, C2H4OC3H7, C2H4OC4H9, C2H4OC6H5, C2H4OC6H11,
C2H4CN, C5H10CN, C6H12CN, C2H4OC2H4CN, C3H6OC2H4CN, C3H6OCH3, C3H6OC2H5

                         C2H5
                         |
C3H6OC3H7, C3H6OC4H9, C3H6OCH2-CH-C4H9, C3H6OC6H11, C3H6OC8H17,

C3H6OCH2C6H5, C3H6OC2H4C6H5, C3H6OC2H4OC6H5, C3H6OC6H5, C3H6OC2H4OH,
C3H6OC4H8OH, C3H6OC2H4OCH3, C3H6OC2H4OC2H5, C3H6OC2H4OCH(CH3)2,
C3H6OC2H4OC4H9, C3H6OC2H4OCH2C6H5, C3H6OC2H4OC6H5, C3H6OC4H8OCH3,
C3H6OC4H8OC2H5, C3H6OC4H8OC4H9, CH-CH2OCH3, CHCH2OC4H9, CH-CH2OC6H5,
                                |           |           |
                                CH3         CH3         CH3

CHCH2OCH2C6H5, CH-C2H4OCH3, CH-CH2-OCH3, CH-CH(OCH3)2, CH2CHOCH3,
|              |            |            |                |
CH3            CH3          C2H5         CH3              CH3

CH2-CH-OC2H5, CH2-CH-OC4H9, CH2-CH-OC6H5 sowie die entsprechenden
    |             |             |
    CH3           CH3           CH3

Reste, in denen die Gruppierungen C2H4O, C3H6O, CH-CH2O und CH2-CHO
                                                |              |
zweimal vorhanden sind.                         CH3            CH3

C3H6OCH-CH2OCH3, C3H6OCHOC2H5, C3H6OCH2CHOCH3 und
     |                |                 |
     CH3              CH3               CH3
```

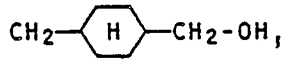

```
                     CH3        CH3             CH3
                     |          |               |
CH2-C6H5, C2H4-C6H5, CH2CH-C6H5, CHC2H4-C6H5, C2H4CH-C6H5, CH2CH-C6H5,
                                                                |
                                                                OH
```

$C_2H_5$ $C_3H_7$

$CH-C_6H_5$, $CH-C_6H_5$ und $C_6H_4CH_3$ und $C_6H_4OCH_3$ anstelle von $C_6H_5$, $(CH_2)_2OCHO$, $(CH_2)_2OCO(CH_2)_nCH_3$, $(C_2H_4O)_2CHO$, $(C_2H_4O)_2CO(CH_2)_nCH_3$, $(CH_2)_3O(CH_2)_2OCHO$, $(CH_2)_3O(CH_2)_2OCO(CH_2)_nCH_3$, $(CH_2)_2O(CH_2)_4OCHO$, $(CH_2)_2O(CH_2)_4OCO(CH_2)_nCH_3$ wobei n die Zahlen 0 bis 7 bedeutet.

$C_2H_5$

$(CH_2)_2OCOCH$ , $(CH_2)_2OCOC_6H_5$, $(CH_2)_2OCOC_6H_4CH_3$,

$C_4H_9$

$(CH_2)_2OCOC_6H_4Cl$, $(CH_2)_2OCONHCH_3$, $(CH_2)_2OCONHC_4H_9$,

$C_2H_5$

$(CH_2)_2OCONHCH_2CH$ , $(CH_2)_2OCONHC_6H_5$, sowie die entsprechenden

$C_4H_9$

Reste mit $(CH_2)_3$ oder $(CH_2)_4$ anstelle von $(CH_2)_2$.

2. Gegebenenfalls substituierte Cycloalklreste:

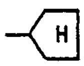 , 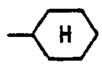 , 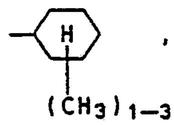 , 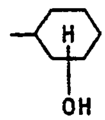 , 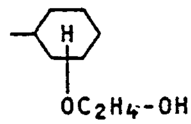 und 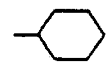 .

3. Gegebenenfalls substituierte Phenylreste: $C_6H_5$, $C_6H_4CH_3$, $C_6H_3(CH_3)_2$, $C_6H_4OCH_3$, $C_6H_3(OCH_3)_2$, $C_6H_4Cl$ und $C_6H_2(OCH_3)_2Cl$.

4. Die Reste: $CH_2CH{=}CH_2$, $CH_2COOCH_3$, $(CH_2)_5COOCH_3$, $(CH_2)_5COOC_2H_5$,

$C_4H_9$ $C_2H_5$

$(CH_2)_5COO$ , $(CH_2)_5COOCH_2CH$ , $(CH_2)_n$ - 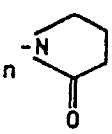 ,

$C_4H_9$

wobei n=2, 3, 4 oder 6 ist.

5. Acylreste: CHO, $CH_3(CH_2)_nCO$ wobei n die Zahlen 0 bis 7 bedeuten kann, $C_6H_5CO$, $CH_3C_6H_4CO$, $C_6H_5CH_2CO$, $C_6H_5OCH_2CO$, $CH_3SO_2$, $C_2H_5SO_2$, $C_6H_5SO_2$ oder $CH_3C_6H_4SO_2$.

Einzelne Reste $R^1$ sind neben den bereits genannten beispielsweise Methyl, Ethyl, Propyl, Butyl, Benzyl, Phenethyl, Phenyl, o-, m-, p-Tolyl oder o-, m-, p-Chlorphenyl.

Reste $R^3$ sind neben den bereits genannten z.B. $C_1$- bis $C_6$-Alkylgruppen, die durch Chlor, Brom, Hydroxy, $C_1$- bis $C_8$-Alkoxy, Phenoxy, Cyan, Carboxy, $C_1$- bis $C_8$-Alkanoyloxy, $C_1$- bis $C_8$-Alkoxy-$C_1$—$C_4$-alkoxy, Benzoyloxy, o-, m-, p-Methylbenzoyloxy, o-, m-, p-Chlorbenzoyloxy, $C_1$- bis $C_8$-Alkoxyalkanoyloxy, Phenoxyalkanoyloxy, $C_1$- bis $C_8$-Alkoxycarbonyloxy, $C_1$- bis $C_8$-Alkoxyalkoxycarbonyloxy, Benzyloxycarbonyloxy, Phenethyloxycarbonyloxy, Phenoxyethoxycarbonyloxy, $C_1$- bis $C_8$-Alkylaminocarbonyloxy, Cyclohexylaminocarbonyloxy, Phenylaminocarbonyloxy, $C_1$- bis $C_8$-Alkoxycarbonyl, $C_1$- bis $C_8$-Alkoxyalkoxycarbonyl, Phenoxycarbonyl, Benzyloxycarbonyl, Phenoxy $C_1$—$C_4$-alkoxy oder Phenethyloxycarbonyl substituiert sein können sowie Phenyl, Benzyl, Phenethyl oder Cyclohexyl.

Einzelne Reste $R^3$ sind z.B. Methyl, Ethyl, Propyl, Butyl, Pentyl, Hexyl, Allyl, Methallyl, 2-Chlorethyl, 2-Bromethyl, 2-Cyanethyl, 2-Hydroxyethyl, 2-Phenyl-2-hydroxyethyl, 2,3-Dihydroxypropyl, 2-Hydroxypropyl, 2-Hydroxybutyl, 2-Hydroxy-3-phenoxypropyl, 2-Hydroxy-3-methoxypropyl, 2-Hydroxy-3-butoxypropyl, 3-Hydroxypropyl, 2-Methoxyethyl, 2-Ethoxyethyl, 2-Propoxyethyl, 2-Butoxyethyl, 2-Phenoxyethyl, 2-Phenoxypropyl, 2-Acetoxyethyl, 2-Propionyloxyethyl, 2-Butyryloxyethyl, 2-Isobutyryloxyethyl, 2-Methoxymethylcarbonyloxyethyl, 2-Ethoxymethylcarbonyloxyethyl, 2-Phenoxymethylcarbonyloxyethyl, 2-Methoxycarbonyloxyethyl, 2-Ethoxycarbonyloxyethyl, 2-Propoxycarbonyloxyethyl, 2-Butoxycarbonyloxyethyl, 2-Phenyloxycarbonyloxyethyl, 2-Benzyloxycarbonyloxyethyl, 2-Methoxyethoxycarbonyloxyethyl, 2-Ethoxyethoxycarbonyloxyethyl, 2-Propoxyethxoycarbonyloxyethyl, 2-Butoxyethoxycarbonyloxyethyl, 2-Methylaminocarbonyloxyethyl, 2-Ethylaminocarbonyloxyethyl, 2-Propylaminocarbonyloxyethyl, 2-Butylaminocarbonyloxyethyl, 2-Methoxycarbonylethyl, 2-Ethoxycarbonylethyl, 2-Propoxycarbonylethyl, 2-Butoxycarbonylethyl, 2-Phenoxycarbonylethyl, 2-Benzyloxycarbonylethyl, 2-β-Phenylethoxycarbonylethyl, 2-Methoxyethoxycarbonylethyl, 2-Ethoxyethoxycarbonylethyl, 2-Propoxyethoxycarbonylethyl, 2-Butoxyethoxycarbonylethyl, 2-Phenoxyethoxycarbonylethyl, oder 2-Benzoylethyl.

Als Reste $R^4$ und $R^5$ kommen beispielsweise Wasserstoff, Methyl, Ethyl, Propyl, Brom, Chlor, Methoxy,

Ethoxy, Phenoxy, Benzyloxy, $C_1$- bis $C_4$-Alkoxycarbonylamino oder gegebenenfalls durch Chlor, Brom, Cyan, Methoxy, Ethoxy oder Phenoxy substituiertes $C_1$- bis $C_6$-Alkanoylamino oder Benzoylamino sowie $C_1$- bis $C_4$-Alkylsulfonylamino oder -Dialkylaminosulfonylamino in Betracht.

Reste $R^6$ sind neben den bereits genannten z.B. Aminocarbonyl, Methylaminocarbonyl, Dimethylaminocarbonyl, Ethylaminocarbonyl, Diethylaminocarbonyl, Methoxycarbonyl, Ethoxycarbonyl, n-, und i-Propoxycarbonyl, n-, i- und sek.-Butoxycarbonyl, Methoxyethoxycarbonyl, Ethoxyethoxycarbonyl, n- und i-Propoxyethoxycarbonyl oder n-, i- und sek.-Butoxyethoxycarbonyl.

Reste $R^7$ sind beispielsweise $C_1$- bis $C_{10}$-Alkyl, Phenyl, Chlor, Brom, ein- oder mehrfach substituiertes Phenyl oder Benzyl.

Die Verbindungen der Formel I haben gelbe bis türkisfarbene Farbtöne und eignen sich insbesondere zum Färben von Polyestern, Polyamiden, Cellulose-estern un Mischgeweben aus Polyestern und Cellulosefasern. Man erhält Färbungen mit in der Regel guten bis sehr guten Echtheiten insbesondere auf Polyestern.

Bei geeigneter Konstitution sind die Farbstoffe reduktiv und/oder alkalisch un reduktiv ätzbar.

Als Ätzmittel kommen dabei die üblicherweise verwendeten Mittel, z.B. Zinn-II-chlorid, Natriumhydrogensulfit, Natriumdithionit oder Hydroxymethansulfonsäure in Betracht. Die Ätzverfahren können ebenfalls wie üblich durchgeführt werden.

Ein Teil der verbindungen der Formel I eignet sich hervorragend zum Färben von Thermoplasten, wie Polystyrol, Polymethacrylat, Polycarbonat, Polyamid und Styrol/Acrylsäure/Butadien-Copolymerisaten, da sie sich durch hohe Farbstärke, Licht- und Temperaturbeständigkeit auszeichnen. Gleichermaßen sind viele erfindungsgemäße Farbstoffe zum Färben und/oder Bedrucken von Polyester-Baumwolle-Mischungen mit hohen Licht- und Waschechtheiten gemäß dem Verfahren der DE—PS 18 11 796 brauchbar.

Die Herstellung der Verbindungen der Formel I kann nach an sich bekannten Methoden erfolgen. Einzelheiten können den Beispielen entnommen werden, in denen sich Angaben über Teile und Prozente, sofern nicht anders vermerkt, auf das Gewicht beziehen.

Von besonderer Bedeutung sind Verbindungen der Formeln I a

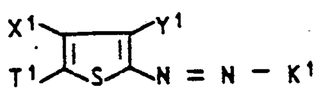

und I b

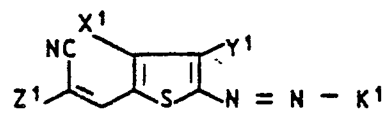

in denen

$X^1$ Chlor, Brom, Hydroxy, $C_1$- bis $C_4$-Alkoxy oder-Alkylthio, Methyl-sufonyl, Phenylsulfonyl, Phenoxy oder Phenylthio,

$Y^1$ Cyan, Carbonester oder substituiertes Carbamoyl

$T^1$ Formyl, Nitro, Cyan,

$Z^1$ Cyan, Carbonester oder substituiertes Carbamoyl und

$K^1$ der Rest einer Kupplungskomponente der Anilin-, Thiazol-, Pyrazol-, Thiophen- oder Pyridinreihe sind.

Technisch besonders wertvoll sind Verbindungen der Formeln Ia und Ib, bei denen

$X^1$ Chlor, Ethoxy oder Phenylmercapto,

$Y^1$ Cyan,

$T^1$ Formyl,

$Z^1$ Cyan, Carbonester oder substituiertes Carbamoyl und

K der Rest einer Kupplungskomponente der Anilin-, Thiazol-, Thiophen- oder Pyridinreihe sind.

Von besonderer Bedeutung sind weiterhin Farbstoffe der allgemeinen Formel IIa

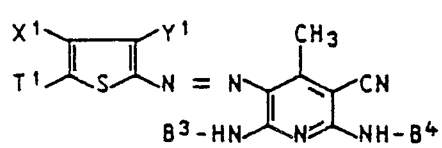

IIa,

in der

$X^1$, $Y^1$ und $T^1$ die angegebene Bedeutung haben und

$B^3$ Wasserstoff oder $B^4$ und

$B^4$ gegebenenfalls durch Sauerstoff unterbrochenes und gegebenenfalls durch Hydroxi, $C_1$- bis $C_4$-Alkanoyloxi, $C_1$- bis $C_4$-Alkoxi, Benzyloxi oder Phenoxi substituiertes $C_2$- bis $C_8$-Alkyl oder gegebenenfalls durch Methyl oder Methoxi substituiertes Phenyl sind.

Bevorzugte Carbonestergruppen $Y^1$ sind $COOCH_3$ oder $COOC_2H_5$.

Für $B^3$ und $B^4$ sind beispielsweise besonders bevorzugt: Wasserstoff und die Reste

$C_2H_5$, $C_3H_7$, $C_4H_9$,

$$CH_2CH \begin{matrix} / C_2H_5 \\ \backslash C_4H_9 \end{matrix}$$

$C_6H_{11}$, $C_6H_5$, $C_6H_4CH_3$, $C_6H_4OCH_3$, $C_2H_4OH$, $C_3H_6OH$, $C_2H_4OCH_3$, $C_3H_6OCH_3$, $C_2H_4OC_2H_5$, $C_2H_4OC_4H_9$, $C_3H_6OC_2H_5$, $C_2H_4OC_2H_4OH$, $C_3H_6OC_2H_4OH$, $C_3H_6OC_4H_8OH$, $C_3H_6OC_2H_4OCH_3$, $C_3H_6OC_2H_4OC_2H_5$, $C_3H_6OC_2H_4OC_4H_9$, $C_3H_6OC_4H_8OC_2H_5$, $C_3H_6OC_4H_8OC_4H_9$, $C_2H_4OC_2H_4OCO(CH_2)_mCH_3$, $C_3H_6OC_2H_4OCO(CH_2)_mCH_3$, $C_3H_6OC_4H_8OCO(CH_2)_mCH_3$, $C_2H_4OCO(CH_2)_mCH_3$, $C_3H_6OCO(CH_2)_mCH_3$, $C_3H_6OCH_2C_6H_5$ und $C_3H_6OC_2H_4OC_6H_5$, wobei m die Zahlen 1—4 bedeutet.

Besonders bevorzugte Kombinationen für $B^3$ und $B^4$ sind z.B.: Wasserstoff und $C_2H_4OC_2OCOCH_3$, $C_2H_4OC_2H_4OCOC_2H_5$, $C_3H_6OC_4H_8OH$, $C_3H_6OC_4H_8OCOCH_3$, $C_3H_6OC_4H_8OCOC_2H_5$, $C_3H_6OC_2H_4OCH_3$, $C_3H_6OC_2H_4OC_2H_5$, $C_3H_6OC_2H_4OC_4H_9$, $C_3H_6OC_4H_8OC_2H_5$, $C_3H_6OC_4H_8OC_4H_9$, $C_3H_6OCH_2C_6H_5$, $C_3H_6OC_2H_4OC_6H_5$ oder $C_3H_6OCH_2—CH(C_2H_5)C_4H_9$ sowie $C_2H_5$, $C_2H_4OCH_3$, $C_3H_6OCH_3$, $CH(CH_3)_2$ oder $C_4H_9$ in Kombination mit $C_2H_4OC_2H_4OCOCH_3$, $C_3H_6OC_4H_8OH$, $C_3H_6OC_4H_8OCHO$, $C_3H_6OC_4H_8OCOCH_3$, $C_3H_6OC_4H_8OCOC_2H_5$, $C_3H_6OC_2H_4OC_4H_9$, $C_3H_6OC_4H_8OC_2H_5$, $C_3H_6OC_4H_8OC_4H_9$ oder $C_3H_6OC_{23}H_4OC_6H_5$ und weiterhin $C_2H_4ON$, $C_3H_6OH$, $C_2H_4OCOCH_3$, $C_2H_4OCOC_2H_5$, $C_3H_6OCOCH_3$ oder $C_3H_6OCOC_2H_5$ kombiniert mit $C_3H_6OC_2H_4OCH_3$, $C_3H_6OC_2H_4OC_2H_5$, $C_3H_6OC_2H_4OC_4H_9$, $C_3H_6OC_4H_8OC_2H_5$, $C_3H_6OC_4H_8OC_4H_9$, $C_6H_4OCH_3$ oder $C_6H_5$.

Weiterhin besonders bevorzugt sind Verbingungen der Formel

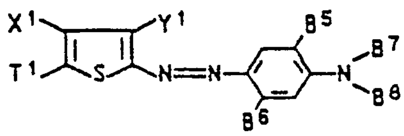

in der

$B^5$ Wasserstoff, Chlor, Methyl, Methoxy oder Ethoxy,

$B^6$ Wasserstoff, Methyl, Methoxy oder $C_1$- bis $C_4$-Alkanoylamino, das noch durch Methoxy, Ethoxy, Phenoxy, Chlor oder Cyan substituiert sein kann,

$B^7$ Wasserstoff oder $B^8$ und

$B^8$ gegebenenfalls durch Hydroxy $C_1$- bis $C_4$-Alkoxy, $C_1$- bis $C_4$-Alkoxycarbonyl, $C_1$- bis $C_4$-Alkanoyloxy oder Cyan substituiertes $C_1$- bis $C_6$-Alkyl, Cyclohexyl, Allyl, Benzyl oder Phenyl,

$X^1$ Chlor, Brom, Hydroxy, $C_1$- bis $C_4$-Alkoxy oder -Alkythio, Methylsulfonyl, Phenylsulfonyl, Phenoxy oder Phenylthio,

$Y^1$ Cyan, Carbonester oder substituiertes Carbamoyl und

$T^1$ Formyl, Nitro oder Cyan bedeuten.

Weiterhin besonders bevorzugt sind Verbindungen der Formel I, bei denen Y Cyan ist.

Weiterhin besonders bevorzugt sind Verbindungen der Formel I, wobei

X Chlor, Methoxy, Ethoxy, Phenylthio, Methylsulfonyl oder Phenylsulfonyl und

T Cyan oder Formyl sind.

Weiterhin besonders bevorzugt sind Verbindungen der Formel I, wobei

X Chlor, Methoxy, Ethoxy, Phenylthio, Methylsulfonyl oder Phenylsulfonyl und

T Nitro sind.

Weiterhin besonders bevorzugt sind Verbindungen der Formel I, wobei

X Chlor, Methoxy, Ethoxy, Phenylthio, Methylsulfonyl oder Phenylsulfonyl und

T ein Rest der Formel CH=B ist, wobei B

$$=C \begin{matrix} / CN \\ \backslash Z \end{matrix}$$

bedeutet und

Z Wasserstoff, Cyan, Carboxyl, Carbonester, gegebenenfalls substituiertes Carbamoyl, Benzimidazolyl, Benzoxazolyl oder Benzthiazolyl ist.

Von besonderer Bedeutung ist auch das Verfahren zur Herstellung der nebenproduktfreien durch Acyloxialkyl- oder Acyloxialkoxialkylreste substituierten 2,6-Diaminopyridine.

Azofarbstoffe mit solchen Kupplungskomponenten mußten bisher durch nachträgliche Acylierung der entsprechenden Hydroxigruppen enthaltenden Farbstoffe in wasserfreien organischen Lösungsmitteln mit Acylhalogeniden oder Säureanhydriden hergestellt werden, da an den Kupplungskomponenten selbst unter diesen Bedingung bevorzugt N-Acylierung erfolgt, die N-Acyl-2,6-diaminopyridine jedoch nicht mehr kupplungsfähig sind.

Erfindungsgemäß erfolgt die Herstellung der Kupplungskomponenten nunmehr durch Zugabe einer wenigstens äquivalenten Menge Schwefelsäure zu einer Lösung oder Suspension des

hydroxygruppenhaltigen substituierten Pyridins in der gewünschten organischen Säure und Rühren bei 20 bis 100°C, vorzugsweise bei 20 bis 50°C. Der Acylierungsgrad ist vom Wassergehalt abhängig und erreicht in wasserfreiem Medium 100%. Wasserfreiheit und 100%iger Umsatz sind jedoch nicht in jedem Fall zur Erzielung optimaler färberischer Eigenschaften notwendig. In den meisten Fällen genügt eine Umsetzungsrate von 70 bis 90%, die Wasserfreiheit nicht voraussetzt und bei der sich die Acylierung vorteilhaft mit der Herstellung des Hydroxialkylaminopyridins verbinden läßt.

In der JP—A—42 376/1984 ist angegeben, daß man durch Umsetzung von Mercaptoessigestern mit Malodinitril Verbindungen der Formel I erhalten würde. Wie jedoch schon aus J. Org. Chem. *38*, 3616 (1973), sowie J. Heterocyclic Chem, *16*, 1541 (1979), hervorgeht, trifft das nicht zu, denn bei diesen Reaktionen entstehen ausschließlich Thiazolderivate.

Weiterhin sind aus der GB—A—1 436 176 sowie aus der FRA—A—2 190 883 Azofarbstoffe bekannt, die Diazokomponenten auf Thiophenbasis aufweisen. Bei denjenigen Farbstoffen, die dabei unter die obengenannte Formel I fallen, waren aber die Diazokomponenten aufgrund der dort gemachten Auguben nicht herstellbar. Bezüglich der Thiophensynthese wird in der GB—A—1 436 176 und der FR—A—2 190 883 u.a. ein Hinweis auf Chem. Ber *98*, 3571 (1965), gegeben. Nach der dort beschriebenen Methode gelingt die Herstellung von 4,5-Dichlorsowie 4,5-Dibrom-2-acetylamino-3-methoxycarbonylthiophen.

Die Research Disclosure 19826/1980 beschreibt in allgemeiner Form Azofarbstoffe, die eine Diazokomponente auf Thiophenbasis und eine Kupplungskomponente auf Anilinbasis aufweisen. Konkrete Farbstoffe werden dabei nicht genannt.

Aus der JP—A—204 658/1984 sind Thiophenazofarbstoffe bekannt, die denen der Formel I ähnlich sind. Es hat sich jedoch gezeigt, daß die dort beschriebenen Farbstoffe Mängel in ihren anwendungstechnischen Eigenschaften aufweisen.

Schließlich sind in der älteren Anmeldung DE—A—3 529 831, zu der äquivalente nationale Anmeldungen in CH/LI, GB, FR und IT existieren, Azofarbstoffe beschrieben, die denen der Formel I. entsprechen.

Beispiel 1

4,7 Teile 2-Amino-4-chlor-3-cyan-5-formyl-thiophen wurden bei maximal 30°C in 30 Vol.-Teilen 85% iger Schwefelsäure angerührt. Nach dem Zutropfen von 8,3 Teilen Nitrosylschwefelsäure (11.5% $N_2O_3$) bei 0—5°C.wurde bei dieser Temperatur 4 Stunden gerührt.

Die so erhaltene Diazoniumsalzlösung ließ man bei 0°C in eine Lösung von 5,8 Teilen N,N-Diallyl-3-amino-acetanilid in einer Mischung aus 25 Vol.-Teilen DMF, 125 Teilen Wasser, 300 Teilen Eis, 20 Vol.-Teilen 32% iger Salzsäure und 0,5 Teilen Amidosulfonsäure langsam einfließen. Nach Beendigung der Kupplung wurde der Farbstoff abgesaugt, neutral gewaschen und getrocknet. Man erhielt 9 Teile des Farbstoffs der Formel

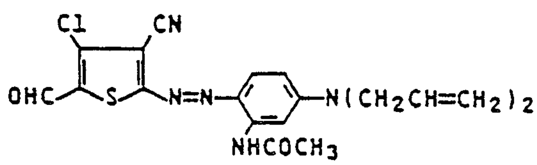

der Polyesterfasern in blauen, echten Nuancen färbt.

Beispiel 2

4,7 Teile 2-Amino-4-chlor-3-cyan-5-formyl-thiophen wurden analog Beispiel 1 diazotiert. Die Diazoniumsalzlösung wurde bei 0°C in eine Lösung von 8,8 Teilen o-(Bisacetoxethyl)amio-p-acetanisidin in einer Mischung aus 125 Teilen Wasser, 350 Teilen Eis. 1 Teil 96% iger Schwefelsäure und 0,5 Teile Amidosulfonsaüre eingetropft. Nach Beendigung der Kupplung wurde der Farbstoff abgesaugt, neutral gewaschen und getrocknet. Man erhielt 9,5 Teile eines grünschwarzen Pulvers der Formel

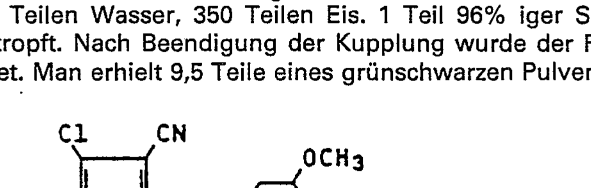

das Polyestergewebe in echten, grünstichig blauen Tönen färbt.

Analog den Beispielen 1—2 erhält man die in der folgenden Tabelle gekennzeichneten Farbstoffe.

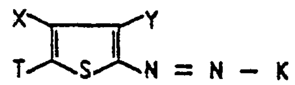

| Bsp. Nr. | T | X | Y | -K | Färbung auf Polyester |
|---|---|---|---|---|---|
| 3 | CHO | Cl | CN | 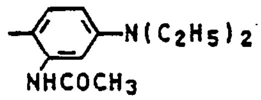 | blau |
| 4 | CHO | Cl | CN | 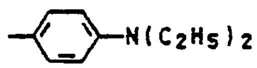 | blauviolett |
| 5 | CHO | Cl | CN | 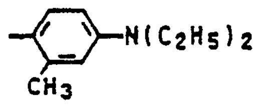 | blau |
| 6 | CHO | Cl | CN | 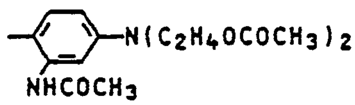 | blau |
| 7 | CHO | Cl | CN | 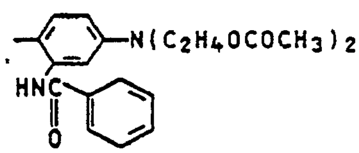 | blau |
| 8 | CHO | Cl | CN | 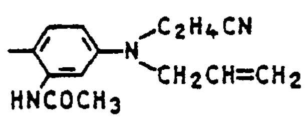 | blau |
| 9 | CHO | Cl | CN | 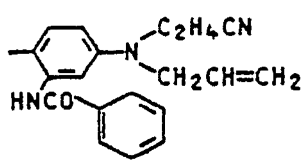 | blau |
| 10 | CHO | Cl | CN | 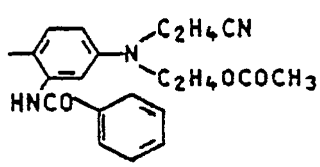 | blau |

| Bsp. Nr. | T | X | Y | –K | Färbung auf Polyester |
|---|---|---|---|---|---|
| 11 | CHO | Cl | CN | 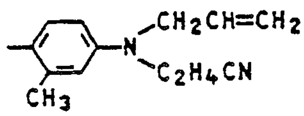 | rotstichig blau |
| 12 | CHO | Cl | CN | 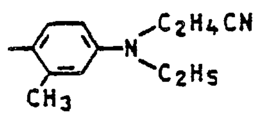 | rotstichig blau |
| 13 | CHO | Cl | CN | 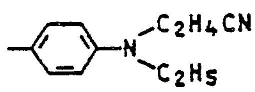 | blauviolett |
| 14 | CHO | Cl | CN | 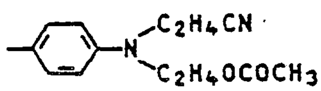 | violett |
| 15 | CHO | Cl | CN | 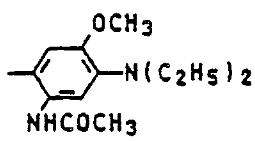 | türkis |
| 16 | CHO | Cl | CN | 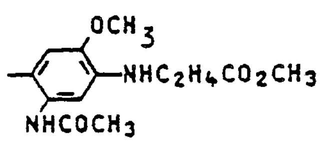 | türkis |
| 17 | CHO | Cl | CN | 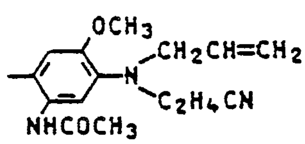 | türkis |
| 18 | CHO | Cl | CN | 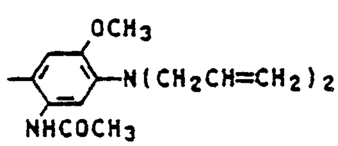 | türkis |
| 19 | CHO | Cl | CN | 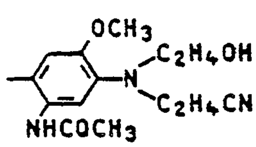 | türkis |
| 20 | CHO | Cl | CN | 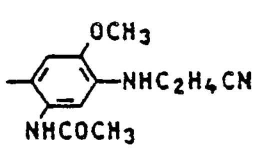 | grünstichig blau |

| Bsp. Nr. | T | X | Y | —K | Färbung auf Polyester |
|---|---|---|---|---|---|
| 21 | CHO | Cl | CN | 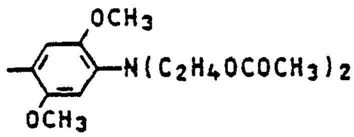 | grünstichig blau |
| 22 | CHO | Cl | CN | 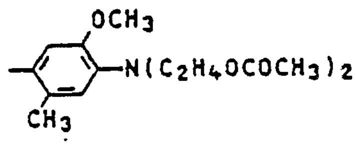 | blau |
| 23 | CHO | Cl | CN | 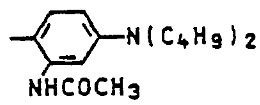 | blau |
| 24 | CHO | Cl | $CO_2C_2H_5$ | 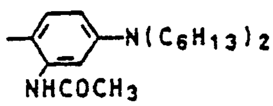 | blau |
| 25 | CHO | Cl | CN | 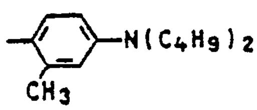 | blau |
| 26 | CHO | Cl | CN | 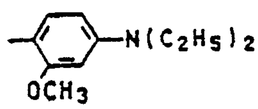 | blau |
| 27 | CHO | Cl | CN | 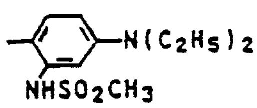 | blau |
| 28 | CHO | Cl | CN | 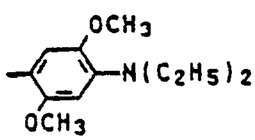 | blau |
| 29 | CHO | Cl | CN | 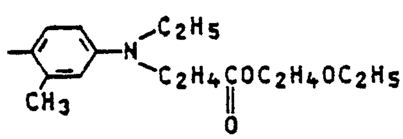 | blau |
| 30 | CHO | Cl | CN | 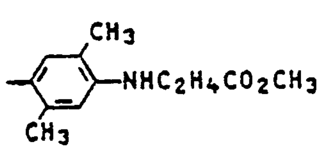 | blau |

| Bsp. Nr. | T | X | Y | –K | Färbung auf Polyester |
|---|---|---|---|---|---|
| 31 | CHO | Cl | $COOCH_3$ | 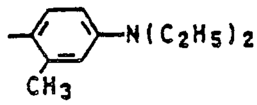 | blau |
| 32 | CHO | Cl | $SO_2CH_3$ | 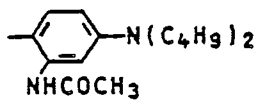 | blau |
| 33 | CHO | Cl | $CONH_2$ | 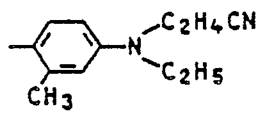 | rotstichig blau |
| 34 | CHO | Cl | $CON(CH_3)_2$ | 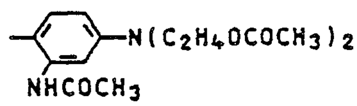 | blau |
| 35 | CHO | Cl | $CO_2C_2H_5$ | 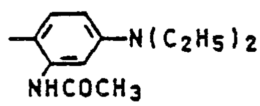 | blau |
| 36 | CHO | Cl | $CO_2C_2H_5$ | 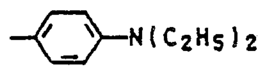 | blauviolett |
| 37 | CHO | Cl | $CO_2C_2H_5$ | 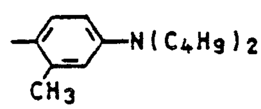 | blau |
| 38 | CHO | Cl | $CO_2C_2H_5$ | 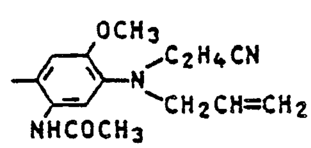 | grünstichig blau |
| 39 | CHO | Br | CN | 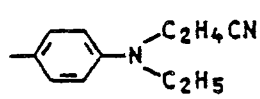 | violett |
| 40 | CHO | Br | CN | 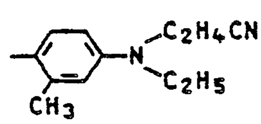 | blauviolett |
| 41 | CHO | Br | CN | 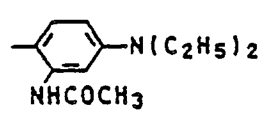 | blau |

| Bsp. Nr. | T | X | Y | —K | Färbung auf Polyester |
|---|---|---|---|---|---|
| 42 | CHO | Br | CN | 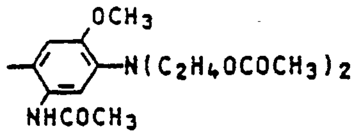 | grünstichig blau |
| 43 | CHO | Br | $CO_2C_2H_5$ | 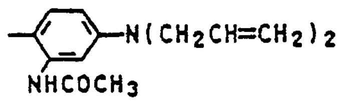 | blau |
| 44 | CHO | F | CN | 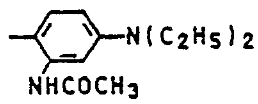 | blau |
| 45 | CHO | F | CN | 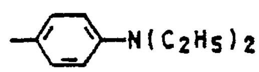 | rotstichig blau |
| 46 | CHO | F | CN | 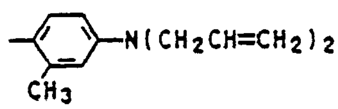 | blau |
| 47 | CHO | F | $CO_2C_2H_5$ | 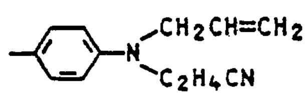 | rotstichig blau |
| 48 | CHO | Cl | $CO_2C_2H_5$ | 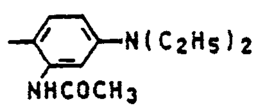 | blau |
| 49 | CHO | Cl | $CONH_2$ | 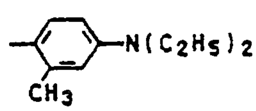 | blau |
| 50 | CHO | Cl | $NO_2$ | 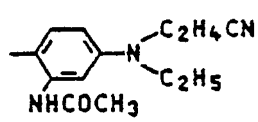 | blau |
| 51 | CHO | Cl | $CON(CH_3)_2$ | 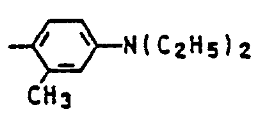 | blau |
| 52 | CHO | Cl | $SO_2CH_3$ | 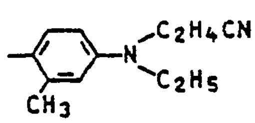 | blau |

| Bsp. Nr. | T | X | Y | —K | Färbung auf Polyester |
|---|---|---|---|---|---|
| 53 | CHO | Br | $CO_2C_2H_5$ | 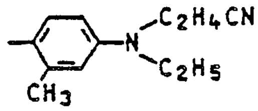 | blau |
| 54 | CHO | Cl | CN | 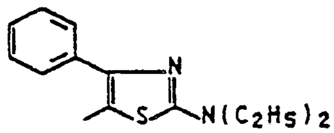 | blau |
| 55 | CHO | Cl | CN | 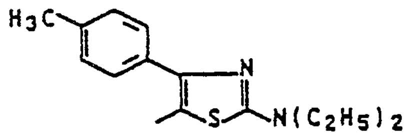 | blau |
| 56 | CHO | Br | CN | 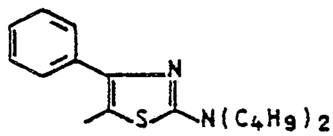 | blau |
| 57 | CHO | Cl | CN | 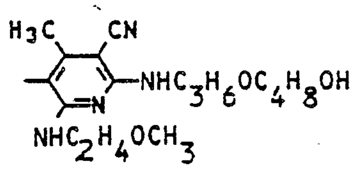 | violett |
| 58 | CHO | Cl | CN | 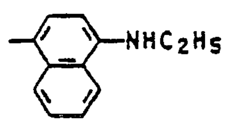 | blau |
| 59 | CHO | Cl | $CO_2C_2H_5$ | 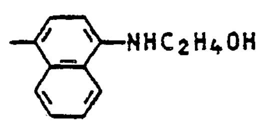 | blau |
| 60 | CHO | Cl | CN | 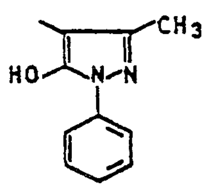 | gelbbraun |
| 61 | CHO | Cl | CN | 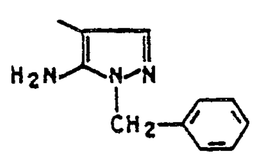 | rot |

| Bsp. Nr. | T | X | Y | -K | Färbung auf Polyester |
|---|---|---|---|---|---|
| 62 | CHO | Cl | CN | 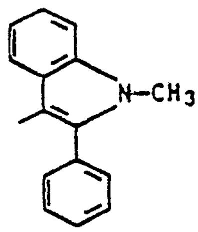 | rot |
| 63 | CHO | Cl | CN | 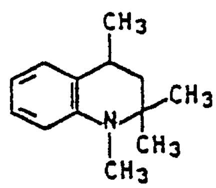 | blau |
| 64 | CHO | Cl | CN | 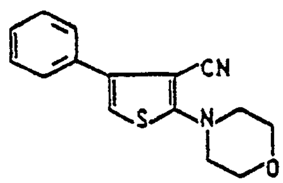 | blau |
| 65 | CHO | Br | CN | 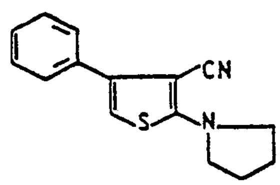 | blau |
| 66 | CHO | Cl | CN | 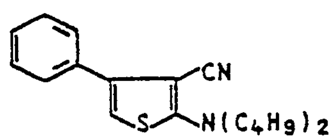 | blau |
| 67 | CHO | Cl | CN | 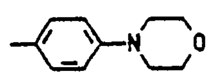 | violett |
| 68 | CHO | Cl | CN | 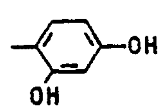 | rot |
| 69 | CHO | Cl | CN | 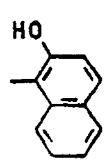 | rot |

| Bsp. Nr. | T | X | Y | —K | Färbung auf Polyester |
|---|---|---|---|---|---|
| 70 | CHO | Cl | CN | 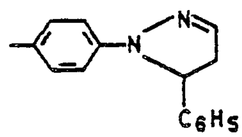 | blau |
| 71 | CHO | Cl | CN | 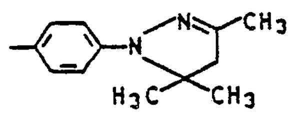 | blau |
| 72 | H | Cl | CN | 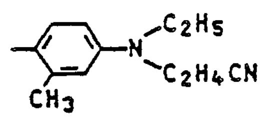 | rot |
| 73 | $CH_3$ | Cl | CN | 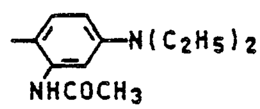 | rot |
| 74 | $CH_3$ | Cl | CN | 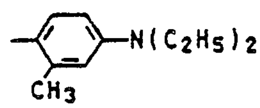 | rot |
| 75 | $CH_3$ | Cl | CN | 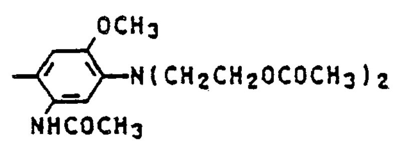 | rotviolett |
| 76 | $C_2H_5$ | Cl | CN | 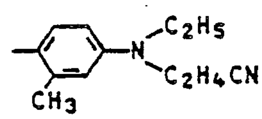 | rot |
| 77 | $CH_3$ | Br | CN | 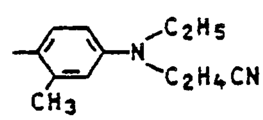 | rot |
| 78 | CN | Cl | CN | 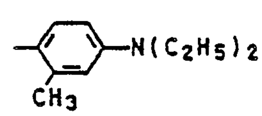 | blau |
| 79 | CN | Cl | CN | 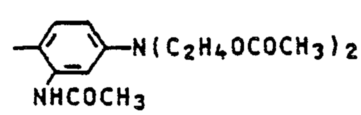 | blau |

| Bsp. Nr. | T | X | Y | -K | Färbung auf Polyester |
|---|---|---|---|---|---|
| 80 | CN | Cl | CN | 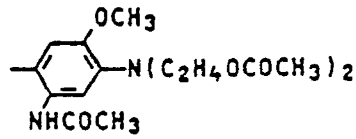 | türkis |
| 81 | CN | Br | CN | 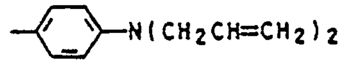 | rotstichig blau |
| 82 | CN | Cl | CN | 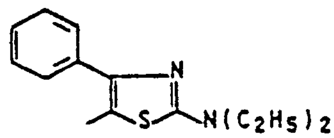 | blau |
| 83 | $NO_2$ | Cl | CN | 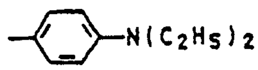 | rotstichig blau |
| 84 | $NO_2$ | Cl | CN | 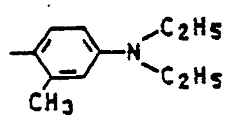 | blau |
| 85 | $NO_2$ | Cl | CN | 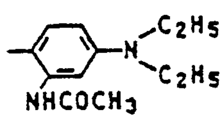 | grünstichig blau |
| 86 | $NO_2$ | Cl | CN | 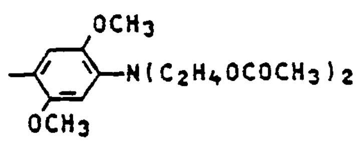 | grünstichig blau |
| 87 | 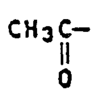 | Cl | CN | 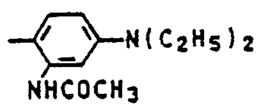 | blau |
| 88 | $NO_2$ | Br | CN | 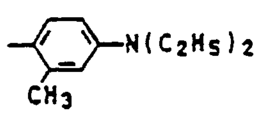 | blau |
| 89 | $NO_2$ | Cl | CN | 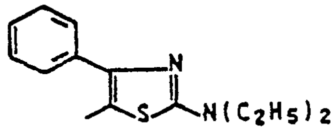 | türkis |
| 90 | $NO_2$ | Cl | CN | 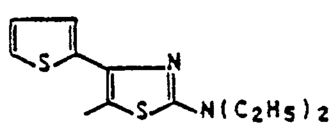 | türkis |

Beispiel 91

11,7 Teile 2-Amino-3-cyan-4-ethoxy-5-formylthiophen wurden bei maximal 20°C in 200 Vol.-Teilen Eisessig/Propionsäure (3:1) und 25 Vol.-Teilen 96% iger Schwefelsäure angerührt. Bei 0—5°C ließ man unter Rühren 23 Teile Nitrosylschwefelsäure (11,5% $N_2O_3$) zutropfen und anschließend 1 Stunde rühren.

Die so erhaltene Diazoniumsalzlösung wurde bei 0°C in eine Lösung von 21 Teilen o-(Bisacetoxethyl)-amino-p-acetanisidin in einer Mischung aus 100 Vol.-Teilen Dimethylformamid, 500 Teilen Wasser, 25 Vol.-Teilen 18% iger Salzsäure, 250 Teilen Eis und 1 Teil amidosulfonsäure getropft. Nach Beendigung der Kupplung wurde der Farbstoff abgesaugt, neutral gewaschen und getrocknet. Man erhielt 25 Teile des Farbstoffs der Formel

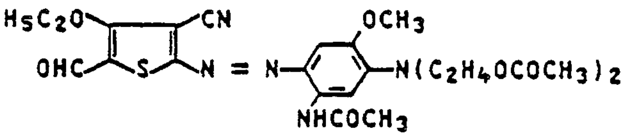

das Polyesterfasern in grünstichig blauen, echten Nuancen anfärbt.

Analog Beispiel 91 wurden die in der folgenden Tabelle angegebenen Farbstoffe erhalten.

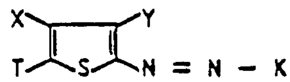

| Bsp. Nr. | T | X | Y | −K | Färbung auf Polyester |
|---|---|---|---|---|---|
| 92 | CHO | $OC_2H_5$ | CN | 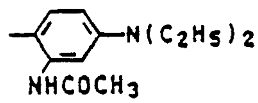 | blau |
| 93 | CHO | $OC_2H_5$ | CN | 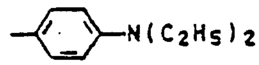 | blauviolett |
| 94 | CHO | $OC_2H_5$ | CN | 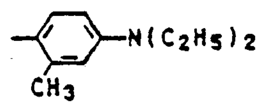 | blau |
| 95 | CHO | $OC_2H_5$ | CN | 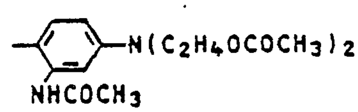 | blau |
| 96 | CHO | $OC_2H_5$ | CN | 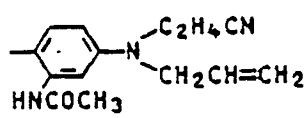 | blau |
| 97 | CHO | $OC_2H_5$ | CN | 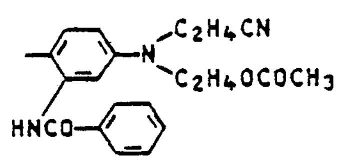 | blau |
| 98 | CHO | $OC_2H_5$ | CN | 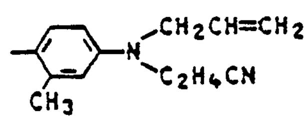 | rotstichig blau |
| 99 | CHO | $OC_2H_5$ | CN | 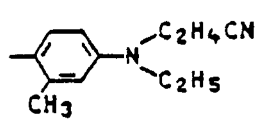 | rotstichig blau |
| 100 | CHO | $OC_2H_5$ | CN | 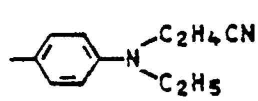 | blauviolett |
| 101 | CHO | $OC_2H_5$ | CN | 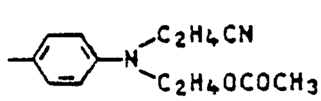 | violett |

| Bsp. Nr. | T | X | Y | —K | Färbung auf Polyester |
|---|---|---|---|---|---|
| 102 | CHO | $OC_2H_5$ | CN | 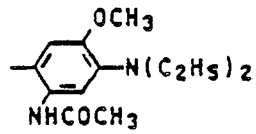 | türkis |
| 103 | CHO | $OC_2H_5$ | CN | 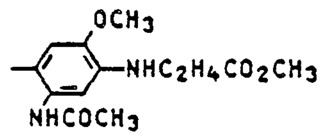 | türkis |
| 104 | CHO | $OC_2H_5$ | CN | 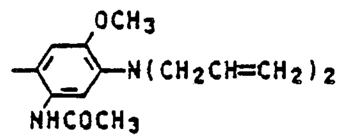 | türkis |
| 105 | CHO | $OC_2H_5$ | CN | 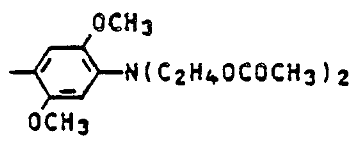 | grünstichig blau |
| 106 | CHO | $OC_2H_5$ | CN | 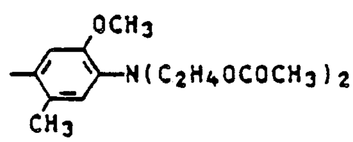 | blau |
| 107 | CHO | $OC_2H_5$ | $CO_2C_2H_5$ | 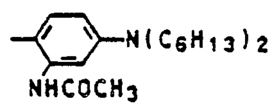 | blau |
| 108 | CHO | $OC_2H_5$ | $COOCH_3$ | 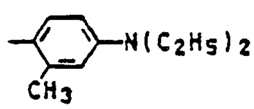 | blau |
| 109 | CHO | $OC_2H_5$ | $SO_2CH_3$ | 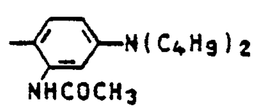 | blau |
| 110 | CHO | $OC_2H_5$ | $CONH_2$ | 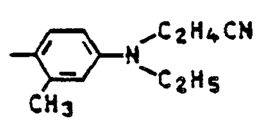 | rotstichig blau |
| 111 | CHO | $OC_2H_5$ | $CON(CH_3)_2$ | 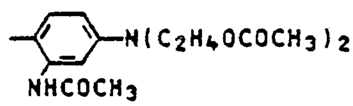 | blau |

| Bsp. Nr. | T | X | Y | −K | Färbung auf Polyester |
|---|---|---|---|---|---|
| 112 | CHO | $OC_2H_5$ | $CO_2C_2H_5$ | 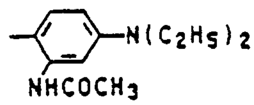 | blau |
| 113 | CHO | $OC_2H_5$ | $CO_2C_2H_5$ | 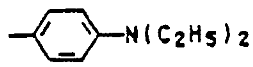 | blauviolett |
| 114 | CHO | $OC_2H_5$ | $CO_2C_2H_5$ | 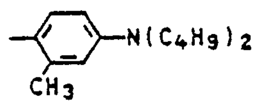 | blau |
| 115 | CHO | $OC_2H_5$ | $CO_2C_2H_5$ | 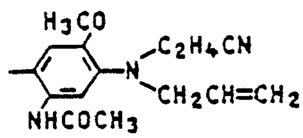 | grünstichig blau |
| 116 | CHO | $OCH_3$ | CN | 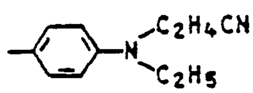 | violett |
| 117 | CHO | $OCH_3$ | CN | 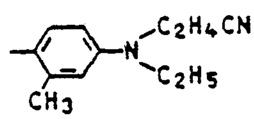 | blauviolett |
| 118 | CHO | $OCH_3$ | CN | 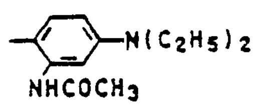 | blau |
| 119 | CHO | $OCH_3$ | CN | 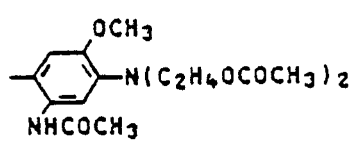 | grünstichig blau |
| 120 | CHO | $OCH_3$ | $CO_2C_2H_5$ | 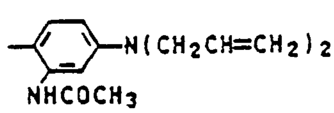 | blau |
| 121 | CHO | $OC_6H_5$ | CN | 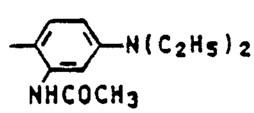 | blau |
| 122 | CHO | $OC_6H_5$ | CN | 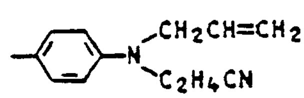 | blauviolett |

| Bsp. Nr. | T | X | Y | –K | Färbung auf Polyester |
|---|---|---|---|---|---|
| 123 | CHO | $SC_6H_5$ | $CO_2C_2H_5$ | 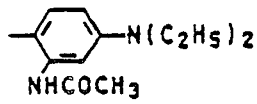 | blau |
| 124 | CHO | $SC_2H_5$ | CN | 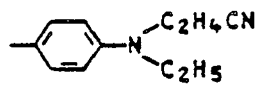 | blauviolett |
| 125 | CHO | $SC_6H_5$ | CN | 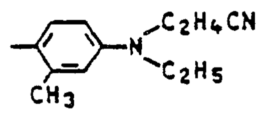 | blau |
| 126 | CHO | $OC_2H_5$ | CN | 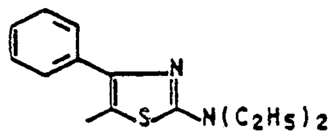 | blau |
| 127 | CHO | $OC_2H_5$ | CN | 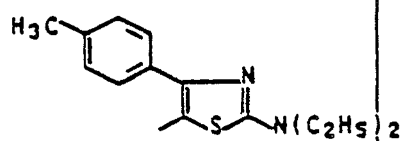 | blau |
| 128 | CHO | $OC_2H_5$ | CN | 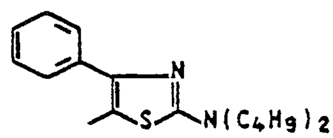 | blau |
| 129 | CHO | $OC_2H_5$ | CN | 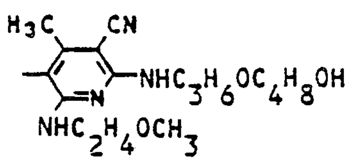 | rotstichig blau |
| 130 | CHO | $OC_2H_5$ | CN | 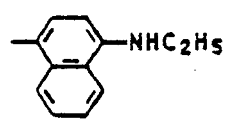 | blau |
| 131 | CHO | $OCH_3$ | $CO_2C_2H_5$ | 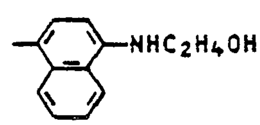 | blau |
| 132 | CHO | $OC_2H_5$ | CN | 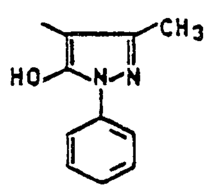 | gelbbraun |

| Bsp. Nr. | T | X | Y | -K | Färbung auf Polyester |
|---|---|---|---|---|---|
| 133 | CHO | $OC_2H_5$ | CN | 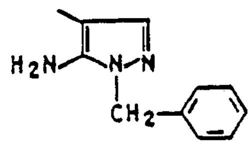 | rot |
| 134 | CHO | $OC_2H_5$ | CN | 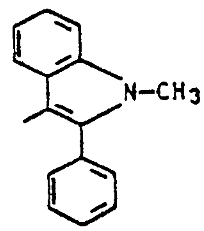 | rot |
| 135 | CHO | $OC_2H_5$ | CN | 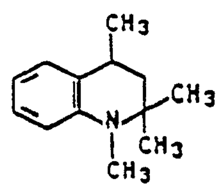 | blau |
| 136 | CHO | $OC_2H_5$ | CN | 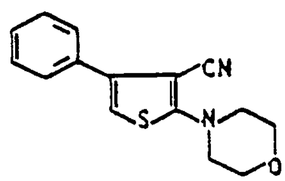 | blau |
| 137 | CHO | $OCH_3$ | CN | 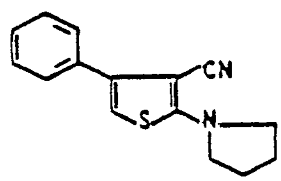 | blau |
| 138 | CHO | $OC_6H_5$ | CN | 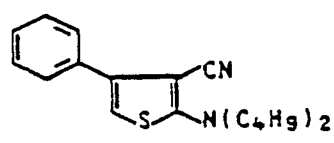 | blau |
| 139 | $CH_3$ | $OC_2H_5$ | CN | 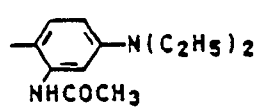 | rot |
| 140 | $CH_3$ | $OC_2H_5$ | CN | 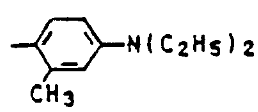 | rot |

| Bsp. Nr. | T | X | Y | -K | Färbung auf Polyester |
|---|---|---|---|---|---|
| 141 | $CH_3$ | $OC_2H_5$ | CN | 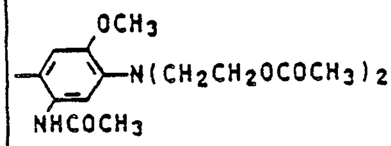 | rotviolett |
| 142 | $C_2H_5$ | $OC_2H_5$ | CN | 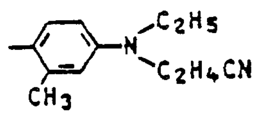 | rot |
| 143 | $CH_3$ | $OC_2H_5$ | CN | 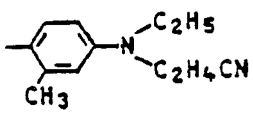 | rot |
| 144 | CN | $OC_2H_5$ | CN | 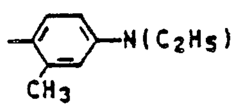 | blau |
| 145 | CN | $OC_2H_5$ | CN | 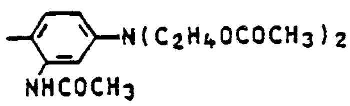 | blau |
| 146 | $NO_2$ | $OC_2H_5$ | CN | 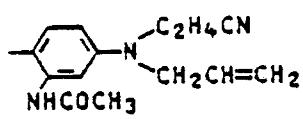 | blau |

Beispiel 147

5,8 Teile des in Beispiel 12 beschriebenen Farbstoffs wurden in 60 Vol.-Teilen Dioxan gelöst, mit 3,4 Teilen Cyanessigsäureethylester, 1 Teil Eisessig und 1 Teil Piperidin versetzt und 16 Stunden bei Raumtemperatur gerührt. Anschließend wurden 50 Teile Wasser und 50 Teile Eis zugegeben. Es wurde 15 Minuten gerührt, das Produkt abgesaugt und neutral gewaschen. Nach dem Trocknen im Vakuum bei 50°C erhielt man 6,6 Teile des Farbstoffs der Formel

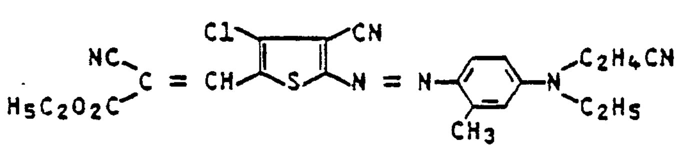

der Polyesterfasern in mittelblauen Tönen färbt.

Beispiel 148

5,1 Teile des in Beispiel 13 beschriebenen Farbstoffs wurden in 65 Volumenteilen Dioxan gelöst, mit 4,2 Teilen Cyanessigsäurebutylester, 1 Teil Eisessig und 1 Teil Piperidin versetzt und 16 Stunden bei Raumtemperatur gerührt. Anschließend wurden 50 Teile Wasser und 50 Teile Eis zugegeben, 1 Stunde gerührt, das Produkt abgesaugt und neutral gewaschen. Nach dem Trocknen wurden 5,9 Teile des Farbstoffs der Formel

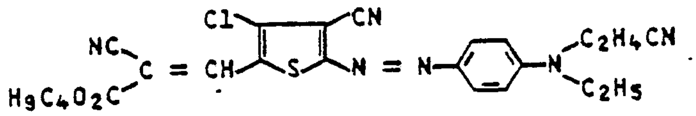

erhalten, der Polyester in echten blauen Tönen färbt.

Analog den Beispielen 147 und 148 wurden die in der folgenden Tabelle aufgeführten Farbstoffe erhalten.

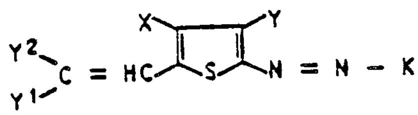

| Bsp. Nr. | X | Y | $Y^1$ | $Y^2$ | −K | Färbung auf PES |
|---|---|---|---|---|---|---|
| 149 | Cl | CN | $COOC_2H_5$ | $COOC_2H_5$ | 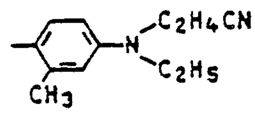 | blau |
| 150 | Cl | CN | $COOC_2H_5$ | $COOC_2H_5$ | 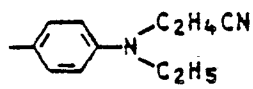 | rot-stichig blau |
| 151 | Cl | CN | $COOC_2H_5$ | $COOC_2H_5$ | 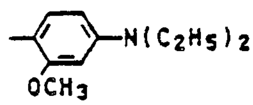 | blau |
| 152 | Cl | CN | $COOC_2H_5$ | $COOC_2H_5$ | 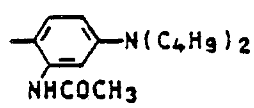 | blau |
| 153 | Cl | CN | CN | CN | 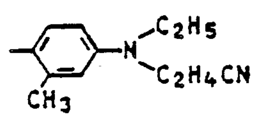 | blau |
| 154 | Cl | CN | $COCH_3$ | $COOC_2H_5$ | 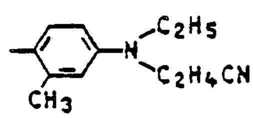 | blau |
| 155 | Cl | $COOC_2H_5$ | CN | $COOC_2H_5$ | 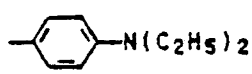 | blau |
| 156 | Cl | $COOC_2H_5$ | CN | $COOC_2H_5$ | 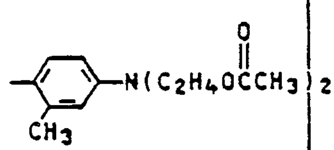 | blau |
| 157 | Cl | $COOC_2H_5$ | CN | $COOC_2H_5$ | 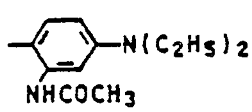 | blau |
| 158 | Cl | $COOC_2H_5$ | $COOC_2H_5$ | $COOC_2H_5$ | 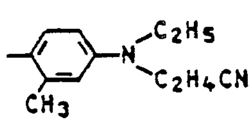 | dunkel-blau |

| Bsp. Nr. | X | Y | $Y^1$ | $Y^2$ | —K | Färbung auf PES |
|---|---|---|---|---|---|---|
| 159 | Cl | $COOC_2H_5$ | $COCH_3$ | $COOC_2H_5$ | 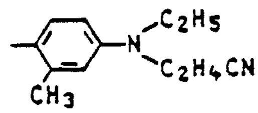 | dunkel-blau |
| 160 | Cl | $COOC_2H_5$ | $COCH_3$ | $COCH_3$ | 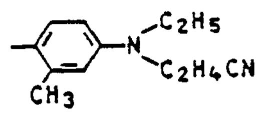 | blau |
| 161 | Cl | $COOC_2H_5$ | $COCH_3$ | CN | 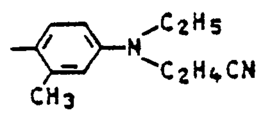 | dunkel-blau |
| 162 | Cl | $C(=O)-NH_2$ | CN | $COOC_2H_5$ | 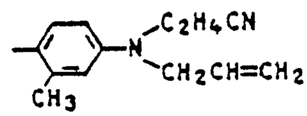 | dunkel-blau |
| 163 | Cl | $C(=O)-NH_2$ | CN | $COOC_2H_5$ | 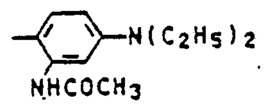 | blau |
| 164 | Cl | $SO_2CH_3$ | CN | $COOC_2H_5$ | 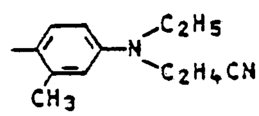 | dunkel-blau |
| 165 | Cl | $SO_2CH_3$ | CN | $COOC_2H_5$ | 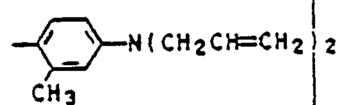 | blau |
| 166 | Cl | $SO_2CH_3$ | CN | $COOC_2H_5$ | 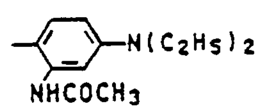 | blau |
| 167 | Cl | CN | CN | $COOC_2H_5$ | 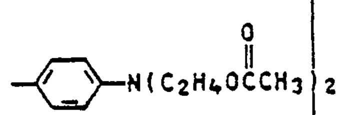 | rot-stichig blau |
| 168 | Cl | CN | CN | $COOC_2H_5$ | 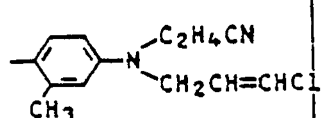 | dunkel-blau |

| Bsp. Nr. | X | Y | Y¹ | Y² | −K | Färbung auf PES |
|---|---|---|---|---|---|---|
| 169 | Cl | CN | CN | $COOC_2H_5$ | 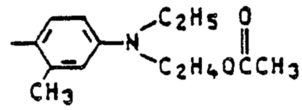 | dunkel-blau |
| 170 | Cl | CN | CN | $COOC_2H_5$ | 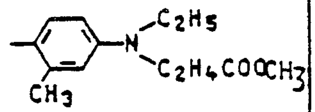 | dunkel-blau |
| 171 | Cl | CN | CN | $COOC_2H_5$ | 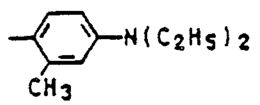 | dunkel-blau |
| 172 | Cl | CN | CN | $COOC_2H_5$ | 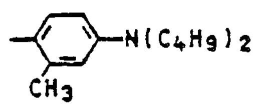 | dunkel-blau |
| 173 | Cl | CN | CN | $COOC_2H_5$ | 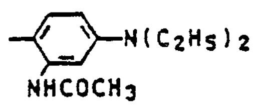 | blau |
| 174 | Cl | CN | CN | $COOC_2H_5$ | 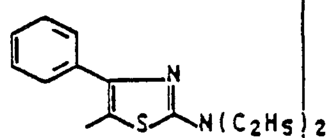 | blau |
| 175 | Cl | CN | CN | $COOC_2H_5$ | 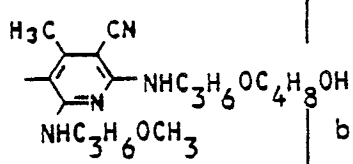 | blau |
| 176 | Cl | CN | CN | $COOC_2H_5$ | 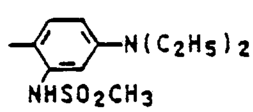 | blau |
| 177 | Cl | CN | CN | $COOC_2H_5$ | 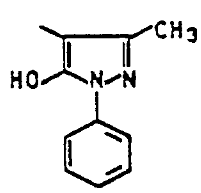 | violett |
| 178 | Cl | CN | CN | $COOC_2H_5$ | 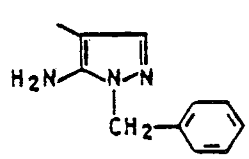 | violett |

| Bsp. Nr. | X | Y | $Y^1$ | $Y^2$ | —K | Färbung auf PES |
|---|---|---|---|---|---|---|
| 179 | Cl | CN | CN | $COOC_2H_5$ | 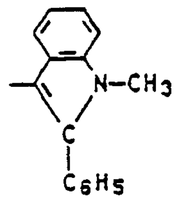 | violett |
| 180 | Cl | CN | CN | $CONHCH_3$ | 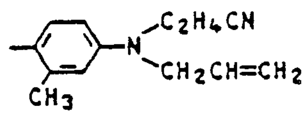 | dunkel-blau |
| 181 | Cl | CN | CN | $CONHC_2H_5$ | 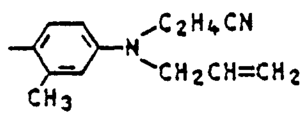 | dunkel-blau |
| 182 | Cl | CN | CN | $SO_2CH_3$ | 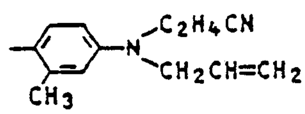 | dunkel-blau |
| 183 | Cl | CN | CN | $COCH_3$ | 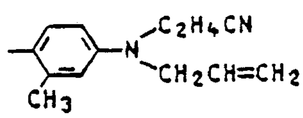 | dunkel-blau |
| 184 | Cl | CN | CN | $COOCH_3$ | 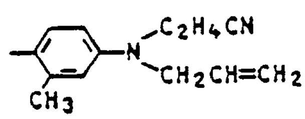 | dunkel-blau |
| 185 | Cl | CN | CN | $COOC_4H_9(n)$ | 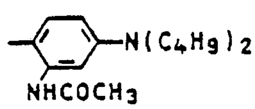 | blau |
| 186 | Cl | CN | CN | $COOC_4H_9(n)$ | 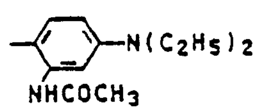 | blau |
| 187 | Cl | CN | CN | $COOC_4H_9(n)$ | 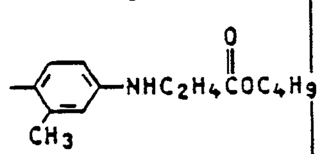 | blau |

| Bsp. Nr. | X | Y | Y¹ | Y² | −K | *) |
|---|---|---|---|---|---|---|
| 188 | Cl | CN | CN | $COOC_4H_9(n)$ | 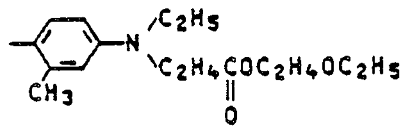 | dunkel-blau |
| 189 | Cl | CN | CN | CO—NH— 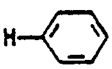 | 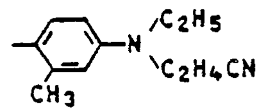 | dunkel-blau |
| 190 | Cl | CN | CN | 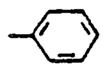 | 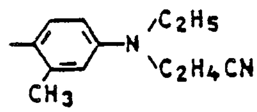 | dunkel-blau |
| 191 | Cl | CN | CN | 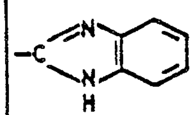 | 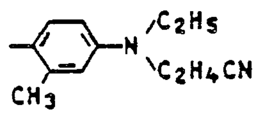 | dunkel-blau |
| 192 | Cl | CN | CN | $CONH_2$ | 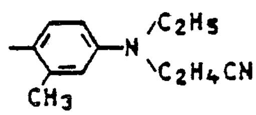 | dunkel-blau |
| 193 | Cl | CN | $COOC_2H_5$ | $COOC_2H_5$ | 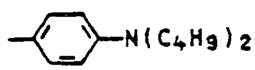 | marine-blau |
| 194 | Cl | CN | $COCH_3$ | $COOC_2H_5$ | 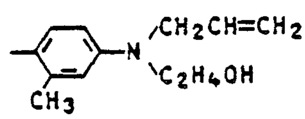 | dunkel-blau |
| 195 | Cl | CN | CN | $CONHCH_3$ | 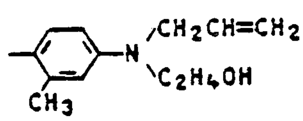 | dunkel-blau |
| 196 | Br | CN | CN | $COOC_2H_5$ | 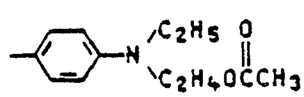 | rot-stichig blau |
| 197 | Br | CN | CN | $COOC_2H_5$ | 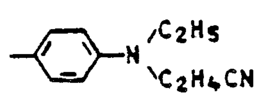 | rot-stichig blau |
| 198 | Br | CN | CN | $COOC_2H_5$ | 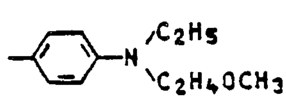 | blau |

*) Färbung auf Polyester

| Bsp. Nr. | X | Y | $Y^1$ | $Y^2$ | −K | *) |
|---|---|---|---|---|---|---|
| 199 | Br | CN | CN | $COOC_2H_5$ | 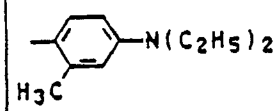 | dunkel-blau |
| 200 | Br | CN | CN | $COOC_2H_5$ | 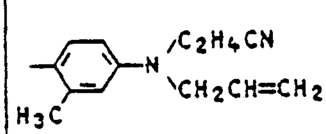 | dunkel-blau |
| 201 | Br | CN | CN | $COOC_2H_5$ | 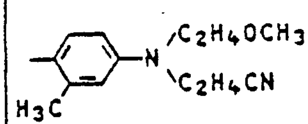 | dunkel-blau |
| 202 | Br | CN | CN | $COOC_2H_5$ | 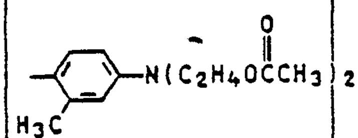 | dunkel-blau |
| 203 | Br | CN | CN | $COOC_2H_5$ | 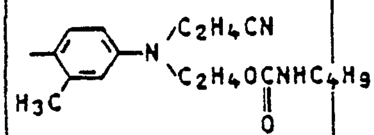 | dunkel-blau |
| 204 | Br | CN | CN | $COOC_2H_5$ | 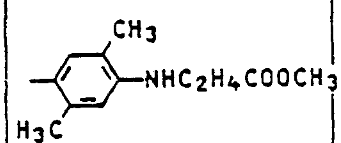 | dunkel-blau |
| 205 | Br | CN | CN | $COOC_4H_9(n)$ | 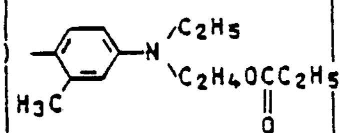 | dunkel-blau |
| 206 | Br | $COOC_2H_5$ | CN | CN | 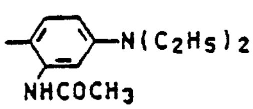 | blau |
| 207 | Br | $COOC_2H_5$ | CN | $COOC_2H_9$ | 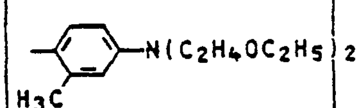 | dunkel-blau |
| 208 | Br | $COOC_2H_5$ | CN | $CON(CH_3)_2$ | 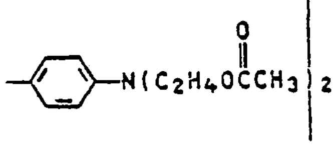 | blau violett |

*) Färbung auf Polyester

| Bsp. Nr. | X | Y | Y¹ | Y² | −K | *) |
|---|---|---|---|---|---|---|
| 209 | Br | $CONH_2$ | CN | $COOC_2H_5$ | 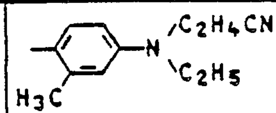 | dunkel-blau |
| 210 | Br | $SO_2CH_3$ | CN | $COOC_2H_5$ | 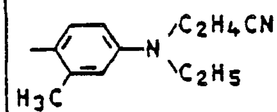 | dunkel-blau |
| 211 | Br | $CON(CH_3)_2$ | CN | $COOC_2H_5$ | 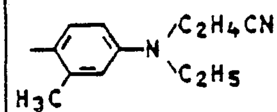 | dunkel-blau |
| 212 | Br | CN | CN | $COOC_2H_5$ | 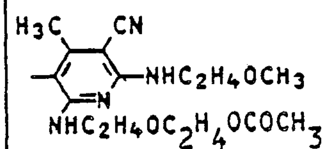 | dunkel-blau |
| 213 | Br | CN | CN | $COOC_2H_5$ | 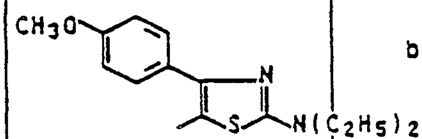 | blau |
| 214 | Br | CN | CN | $COOCH_3$ | 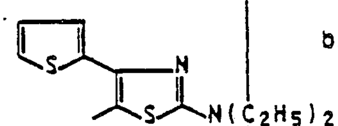 | blau |
| 215 | Br | CN | CN | $COOCH_3$ | 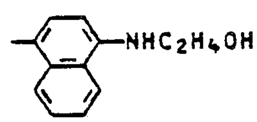 | marine-blau |
| 216 | Cl | CN | CN | 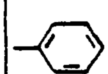 | 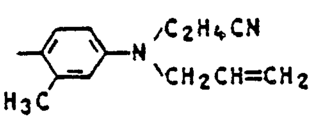 | dunkel-blau |
| 217 | F | CN | CN | $COOC_2H_5$ | 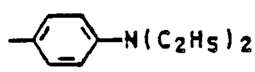 | dunkel-blau |
| 218 | F | CN | CN | $COOC_2H_5$ | 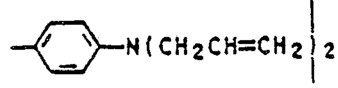 | dunkel-blau |

*) Färbung auf Polyester

| Bsp. Nr. | X | Y | $Y^1$ | $Y^2$ | -K | *) |
|---|---|---|---|---|---|---|
| 219 | F | CN | CN | $COOC_2H_5$ | 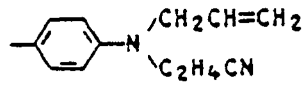 | dunkel-blau |
| 220 | F | $COOC_2H_5$ | CN | $COOC_2H_5$ | 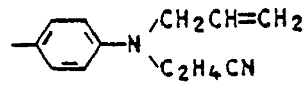 | dunkel-blau |
| 221 | $OC_2H_5$ | CN | $COOC_2H_5$ | $COOC_2H_5$ | 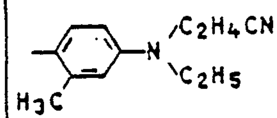 | dunkel-blau |
| 222 | $OC_2H_5$ | CN | $COOC_2H_5$ | $COOC_2H_5$ | 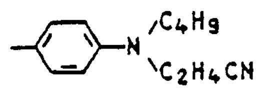 | blau |
| 223 | $OC_2H_5$ | CN | $COOC_2H_5$ | $COOC_2H_5$ | 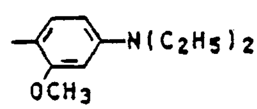 | dunkel-blau |
| 224 | $OC_2H_5$ | CN | $COOC_2H_5$ | $COOC_2H_5$ | 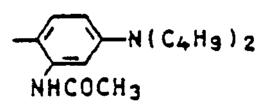 | blau |
| 225 | $OC_2H_5$ | CN | CN | CN | 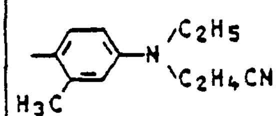 | dunkel-blau |
| 226 | $OC_2H_5$ | CN | $COCH_3$ | $COOC_2H_5$ | 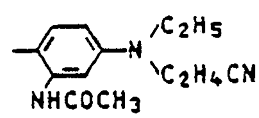 | dunkel-blau |
| 227 | $OC_2H_5$ | $COOC_2H_5$ | CN | $COOC_2H_5$ | 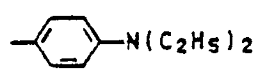 | blau |
| 228 | $OC_2H_5$ | $COOC_2H_5$ | CN | $COOC_2H_5$ | 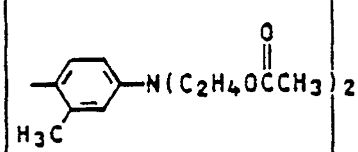 | dunkel-blau |
| 229 | $OC_2H_5$ | $COOC_2H_5$ | CN | $COOC_2H_5$ | 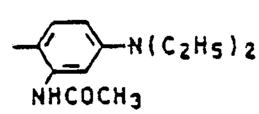 | blau |

*) Färbung auf Polyester

| Bsp. Nr. | X | Y | $Y^1$ | $Y^2$ | −K | *) |
|---|---|---|---|---|---|---|
| 230 | $OC_2H_5$ | $COOC_2H_5$ | $COOC_2H_5$ | $COOC_2H_5$ | 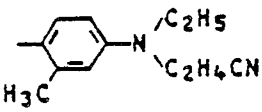 | dunkel-blau |
| 231 | $OC_2H_5$ | $COOC_2H_5$ | $COCH_3$ | $COOC_2H_5$ | 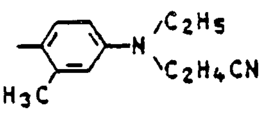 | dunkel-blau |
| 232 | $OC_2H_5$ | $COOC_2H_5$ | $COCH_3$ | $COCH_3$ | 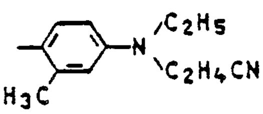 | blau-violett |
| 233 | $OC_2H_5$ | $COOC_2H_5$ | $COCH_3$ | CN | 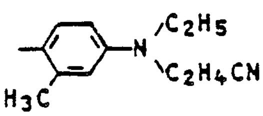 | dunkel-blau |
| 234 | $OC_2H_5$ | 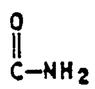 | CN | $COOC_2H_5$ | 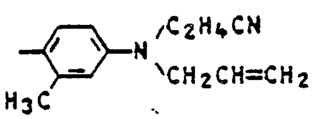 | dunkel-blau |
| 235 | $OC_2H_5$ | 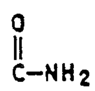 | CN | $COOC_2H_5$ | 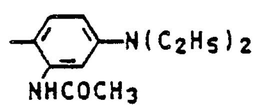 | blau |
| 236 | $OC_2H_5$ | $SO_2CH_3$ | CN | $COOC_2H_5$ | 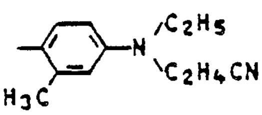 | dunkel-blau |
| 237 | $OC_2H_5$ | $SO_2CH_3$ | CN | $COOC_2H_5$ | 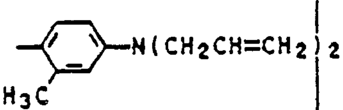 | dunkel-blau |
| 238 | $OC_2H_5$ | $SO_2CH_3$ | CN | $COOC_2H_5$ | 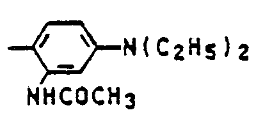 | blau |
| 239 | $OC_2H_5$ | CN | CN | $CON(CH_3)_2$ | 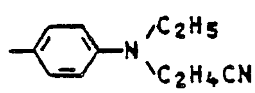 | rotstichig blau |
| 240 | $OC_2H_5$ | CN | CN | $COOC_2H_5$ | 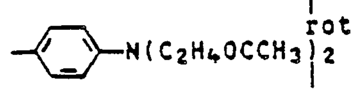 | rotstichig blau |

*) Färbung auf Polyester

| Bsp. Nr. | X | Y | Y¹ | Y² | −K | *) |
|---|---|---|---|---|---|---|
| 241 | $OC_2H_5$ | CN | CN | $COOC_2H_5$ | 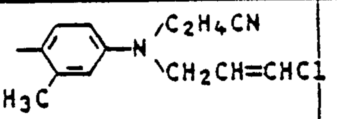 | dunkel-blau |
| 242 | $OC_2H_5$ | CN | CN | $COOC_2H_5$ | 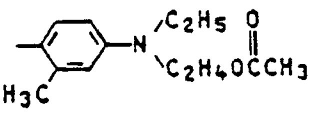 | dunkel-blau |
| 243 | $OC_2H_5$ | CN | CN | $COOC_2H_5$ | 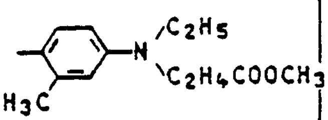 | dunkel-blau |
| 244 | $OC_2H_5$ | CN | CN | $COOC_2H_5$ | 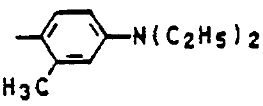 | dunkel-blau |
| 245 | $OC_2H_5$ | CN | CN | $COOC_2H_5$ | 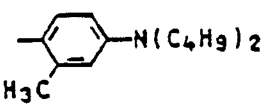 | dunkel-blau |
| 246 | $OC_2H_5$ | CN | CN | $COOC_2H_5$ | 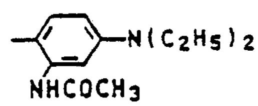 | blau |
| 247 | $OC_2H_5$ | CN | CN | $COOC_2H_5$ | 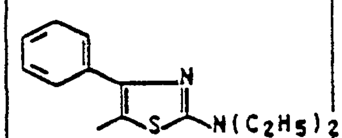 | blau |
| 248 | $OC_2H_5$ | CN | CN | $COOC_2H_5$ | 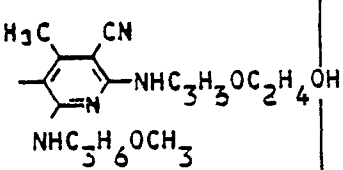 | blau |
| 249 | $OC_2H_5$ | CN | CN | $COOC_4H_9(n)$ | 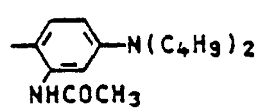 | blau |
| 250 | $OC_2H_5$ | CN | CN | $COOC_4H_9(n)$ | 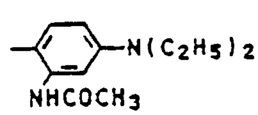 | blau |

*) Färbung auf Polyester

| Bsp. Nr. | X | Y | Y¹ | Y² | —K | *) |
|---|---|---|---|---|---|---|
| 251 | $OC_2H_5$ | CN | CN | $COOC_4H_9(n)$ | 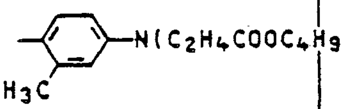 | blau |
| 252 | $OC_2H_5$ | CN | CN | $COOC_4H_9(n)$ | 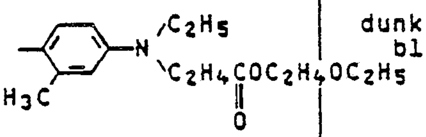 | dunkel-blau |
| 253 | $OC_2H_5$ | CN | CN | 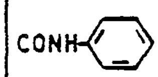 | 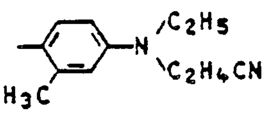 | dunkel-blau |
| 254 | $OC_2H_5$ | CN | CN | 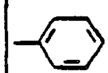 | 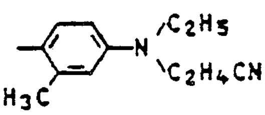 | dunkel-blau |
| 255 | $OC_2H_5$ | CN | CN | 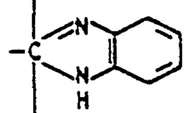 | 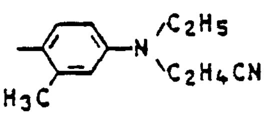 | dunkel-blau |
| 256 | $OC_2H_5$ | CN | CN | $CONH_2$ | 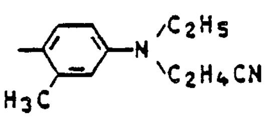 | dunkel-blau |
| 257 | $OC_2H_5$ | CN | $COOC_2H_5$ | $COOC_2H_5$ | 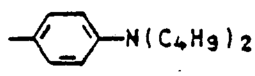 | marine-blau |
| 258 | $OC_2H_5$ | CN | $COCH_3$ | $COOC_2H_5$ | 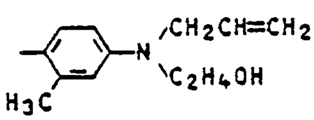 | dunkel-blau |
| 259 | $OC_2H_5$ | CN | CN | $CONHCH_3$ | 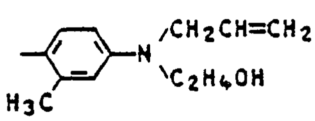 | dunkel-blau |
| 260 | $OC_2H_5$ | CN | CN | $COOC_2H_5$ | 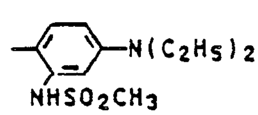 | marine-blau |

*) Färbung auf Polyester

| Bsp. Nr. | X | Y | $Y^1$ | $Y^2$ | −K | *) |
|---|---|---|---|---|---|---|
| 261 | $OC_2H_5$ | CN | CN | $COOC_2H_5$ | 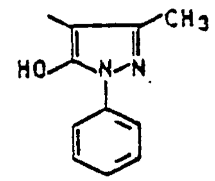 | violett |
| 262 | $OC_2H_5$ | CN | CN | $COOC_2H_5$ | 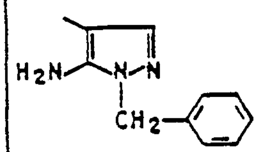 | violett |
| 263 | $OC_2H_5$ | CN | CN | $COOC_2H_5$ | 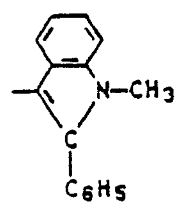 | violett |
| 264 | $OC_2H_5$ | CN | CN | $CONHCH_3$ | 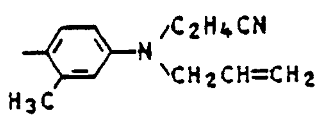 | dunkel-blau |
| 265 | $OC_2H_5$ | CN | CN | $CONHC_2H_5$ | 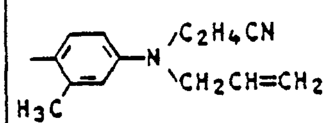 | dunkel-blau |
| 266 | $OC_2H_5$ | CN | CN | $SO_2CH_3$ | 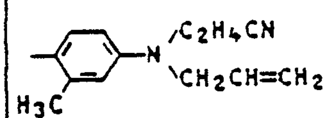 | dunkel-blau |
| 267 | $OC_2H_5$ | CN | CN | $COCH_3$ | 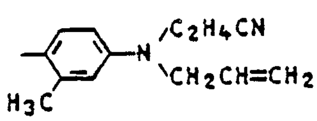 | dunkel-blau |
| 268 | $OC_2H_5$ | CN | CN | $COOCH_3$ | 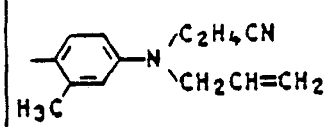 | dunkel-blau |
| 269 | $OCH_3$ | CN | CN | $COOC_2H_5$ | 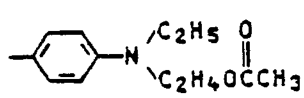 | blau-violett |

*) Färbung auf Polyester

| Bsp. Nr. | X | Y | $Y^1$ | $Y^2$ | –K | *) |
|---|---|---|---|---|---|---|
| 270 | $OC_6H_5$ | CN | CN | $COOC_2H_5$ | 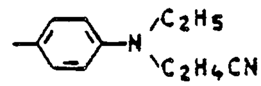 | blau-violett |
| 271 | $SC_6H_5$ | CN | CN | $COOC_2H_5$ | 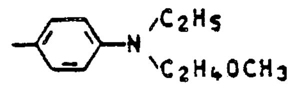 | blau-violett |
| 272 | $SC_6H_5$ | CN | CN | $COOC_2H_5$ | 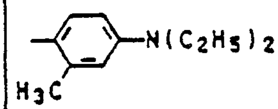 | dunkel-blau |
| 273 | $OCH_3$ | CN | CN | $COOC_2H_5$ | 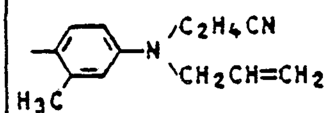 | dunkel-blau |
| 274 | $OCH_3$ | CN | CN | $COOC_2H_5$ | 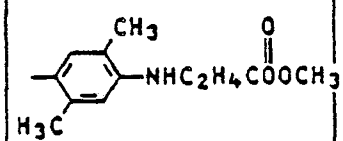 | blau |
| 275 | $OCH_3$ | CN | CN | $COOC_4H_9(n)$ | 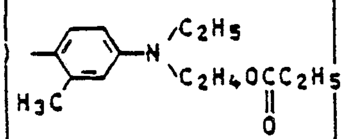 | blau |
| 276 | $OC_6H_5$ | CN | CN | $COOC_2H_5$ | 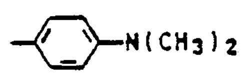 | dunkel-blau |
| 277 | $SC_6H_5$ | CN | CN | $COOC_2H_5$ | 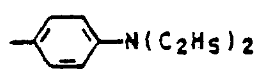 | dunkel-blau |
| 278 | $SC_6H_5$ | CN | CN | $COOC_2H_5$ | 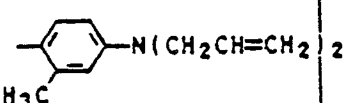 | dunkel-blau |
| 279 | $SC_6H_5$ | CN | CN | $COOC_2H_5$ | 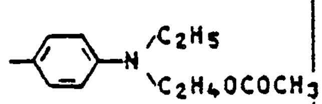 | dunkel-blau |
| 280 | $SC_2H_5$ | CN | CN | $COOC_2H_5$ | 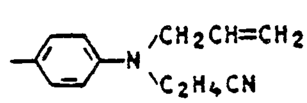 | dunkel-blau |
| 281 | $SC_2H_5$ | $COOC_2H_5$ | CN | $COOC_2H_5$ | 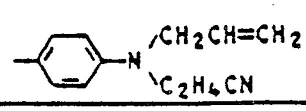 | dunkel-blau |

*) Färbung auf Polyester

Beispiel 282

a) 18,6 Teile 2-Amino-4-chlor-3-cyan-5-formyl-thiophen wurden in 140 Volumenteilen Ethanol suspendiert und mit 2 Teilen Eisessig sowie 2 Teilen Piperidin versetzt. Anschließend ließ man bei Raumtemperatur 50 Volumenteile Cyanessigsäureethylester zutropfen und rührte 7 Stunden bei 60°C. Der Ansatz wurde in 500 Teile einer Eis/Wassermischung eingerührt, der Niederschlag abgesaugt, mit Wasser gewaschen und bei 60°C im Vakuum getrocknet. Man erhielt 20 Teile 2-Amino-4-chlor-3-cyan-5-(β-cyan-β-carboethoxy)-vinyl)-thiophen der Formel

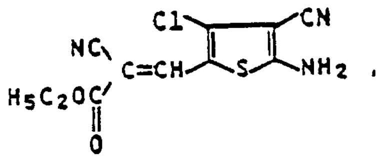

das ohne weitere Reinigung umgesetzt wird.

b) 14 Teile 2-Amino-4-chlor-3-cyan-5-(μ-cyan-β-carboxyethoxy)-vinyl)-thiophen wurden in 100 Volumenteilen Phosphorsäure 85% suspendiert und bei 0—5°C langsam mit 16 Teilen Nitrosylschwefelsäure (11,5% $N_2O_3$) versetzt. Nach 2 h bei 0—5°C ließ man die Diazoniumsalzlösung in eine Lösung von 9,75 Teilen N-Cyanethyl-N-ethyl-m-toluidin in einer Mischung aus 125 Teilen Wasser, 500 Teilen Eis, 25 Volumenteilen 32%iger Salzsäure und 1 Teil Amidosulfonsäure einfließen. Nach Beendigung der Kupplung wurde der Farbstoff abgesaugt, neutral gewaschen und im Vakuum getrocknet. Man erheilt 20 Teile des in Beispiel 147 beschriebenen Farbstoffs der Formel

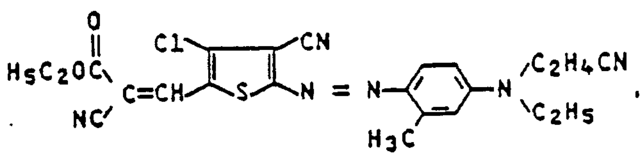

der auf Polyester dunkelblaue Färbungen mit allgemein guten Echtheiten ergibt.

Beispiel 283

14,5 Teile 2-Amino-3-cyan-4-ethoxy-5-((β-cyan-β-carboethoxy)-vinyl)-thiophen wurden in 160 Volumenteilen Phosphorsäure 85% suspendiert und bei 0—5°C mit 16 Teilen Nitrosylschwefelsäure (11,5% $N_2O_3$) diazotiert. Nach 2 h bei 0—5°C setzt man die Diazoniumsalzlösung analog Beispiel 282 mit 9,75 Teilen N-Cyanethyl-N-ethyl-m-toluidin zu 20 Teilen des Farbstoffs der Formel

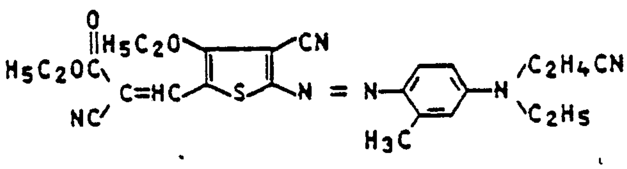

um, mit dem auf Polyester blaue Färbungen mit guter Licht- und Thermofixierechtheit erhalten wurden.

Beispiel 284

14 Teile 2-Amino-4-chlor-3-cyan-5-((β-cyan-β-methylaminocarbonyl)-vinyl)-thiophen wurden in 120 Volumenteilen Phosphorsäure 85% suspendiert, bei 0—5°C mit 16 Teilen Nitrosylschwefelsäure umgesetzt und 2 h bei 0—5°C gerührt. Die Diazoniumsalzlösung ließ man in eine Mischung aus 7,5 Teilen N,N-Diethylanilin, 100 Teilen Wasser, 300 Teilen Eis, 25 Volumenteilen 32%iger Salzsäure und 1 Teil Amidosulfonsäure langsam einfließen. Nach beendeter Kupplung wurde die Suspension filtriert, neutral gewaschen und getrocknet. Man erhielt 16 Teile des Farbstoffs der Formel

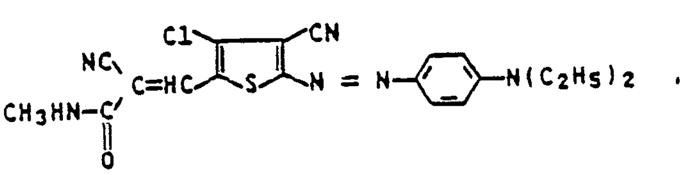

der auf Polyester dunkelblaue Färbungen mit allgemein guten Echtheiten ergibt.

Analog den Beispielen 282—284 wurden auch die in der nachfolgenden Tabelle gekennzeichneten Verbindungen erhalten.

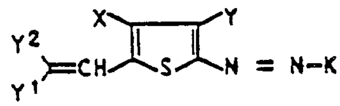

| Bsp. Nr. | X | Y | $Y^1$ | $Y^2$ | –K | *) |
|---|---|---|---|---|---|---|
| 285 | Br | CN | CN | $COOCH_3$ | 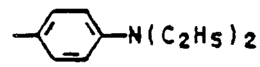 | dunkel-blau |
| 286 | Br | CN | CN | $COOCH_3$ | 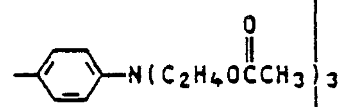 | dunkel-blau |
| 287 | Br | CN | CN | $COOCH_3$ | 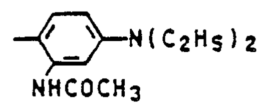 | blau |
| 288 | Br | CN | CN | $COOCH_3$ | 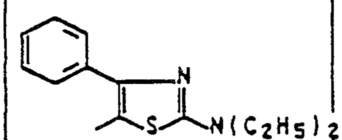 | blau |
| 289 | Cl | CN | CN | $COOC_2H_5$ | 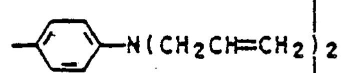 | dunkel-blau |
| 290 | Cl | CN | CN | $COOC_2H_5$ | 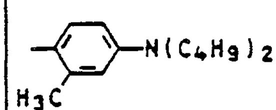 | dunkel-blau |
| 291 | Cl | CN | CN | $COOC_2H_5$ | 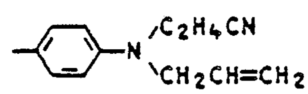 | dunkel-blau |
| 292 | Cl | CN | CN | $COOC_2H_5$ | 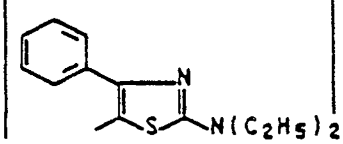 | dunkel-blau |

*) Färbung auf Polyester

| Bsp. Nr. | X | Y | $Y^1$ | $Y^2$ | -K | *) |
|---|---|---|---|---|---|---|
| 293 | Cl | CN | CN | $CONHCH_3$ | 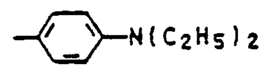 | dunkel-blau |
| 294 | Cl | $COOCH_3$ | CN | $COOC_2H_5$ | 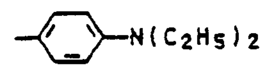 | dunkel-blau |
| 295 | Cl | $SO_2CH_3$ | CN | $COOC_2H_5$ | 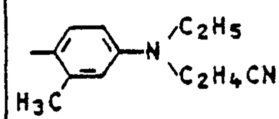 | dunkel-blau |
| 296 | Cl | CN | CN | $COOC_2H_5$ | 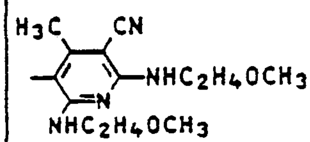 | rotstichig blau |
| 297 | F | CN | CN | $COOC_2H_5$ | 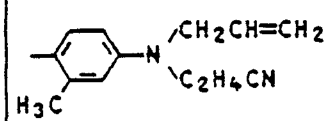 | dunkel-blau |
| 298 | F | CN | CN | $COOC_2H_5$ | 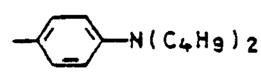 | dunkel-blau |
| 299 | F | CN | CN | $COOC_2H_5$ | 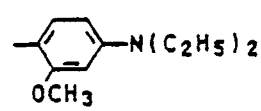 | blau |
| 300 | Cl | CN | CN | CN | 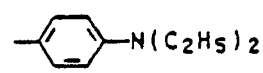 | blau |
| 301 | $OC_2H_5$ | CN | CN | $COOCH_3$ | 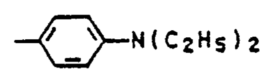 | dunkel-blau |
| 302 | $OC_2H_5$ | CN | CN | $COOCH_3$ | 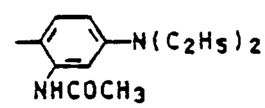 | blau |
| 303 | $OCH_3$ | CN | CN | $COOC_2H_5$ | 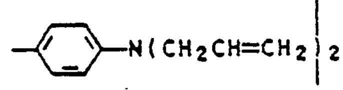 | dunkel-blau |

*) Färbung auf Polyester

| Bsp. Nr. | X | Y | $Y^1$ | $Y^2$ | −K | *) |
|---|---|---|---|---|---|---|
| 304 | $OC_2H_5$ | CN | CN | $COOC_2H_5$ | 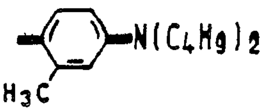 | dunkel-blau |
| 305 | $SC_6H_5$ | CN | CN | $COOC_2H_5$ | 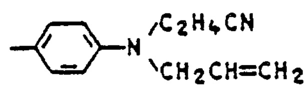 | dunkel-blau |
| 306 | $OC_2H_5$ | CN | CN | $COOCH_3$ | 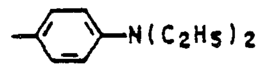 | dunkel-blau |
| 307 | $SC_6H_5$ | CN | CN | $COOC_2H_5$ | 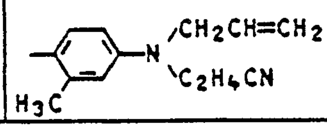 | dunkel-blau |

*) Färbung auf Polyester

Analog zu den beschriebenen Methoden können auch die folgenden durch die Substituenten gekennzeichneten Farbstoffe hergestellt werden:

| Bsp. Nr. | T | X | Y | -K | Farbe auf PES |
|---|---|---|---|---|---|
| 308 | CHO | Cl | CN | 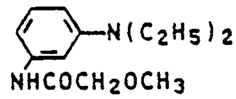 | blau |
| 309 | CHO | Cl | CN | 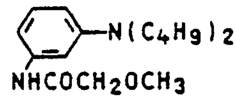 | grünst. blau |
| 310 | CHO | Cl | CN | 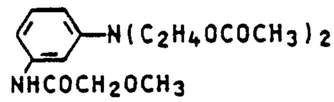 | blau |
| 311 | CHO | Cl | CN | 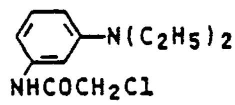 | blau |
| 312 | CHO | Cl | CN | 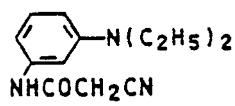 | blau |
| 313 | CHO | Cl | CN | 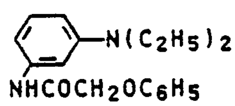 | balu |
| 314 | CHO | Br | CN | 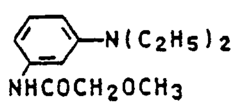 | blau |
| 315 | CHO | Cl | CN | 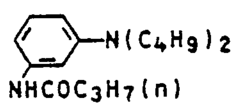 | blau |
| 316 | CHO | Cl | CN | 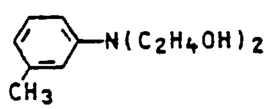 | blau |
| 317 | CHO | Cl | CN | 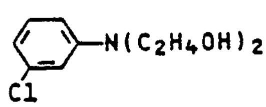 | rotst. blau |

| Bsp. Nr. | T | X | Y | -K | Farbe auf PES |
|---|---|---|---|---|---|
| 318 | CHO | Cl | CN | 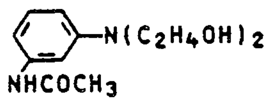 | blau |
| 319 | CHO | Cl | CN | 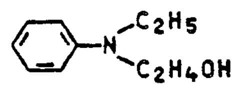 | rotst. blau |
| 320 | CHO | Cl | CN | 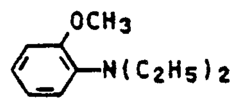 | blau |
| 321 | CHO | Cl | CN | 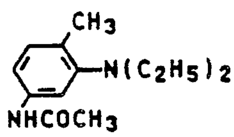 | blau |
| 322 | CHO | Cl | CN | 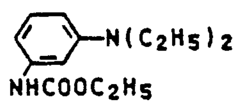 | blau |
| 323 | CHO | Cl | CN | 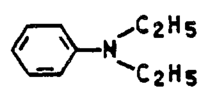 | blau |
| 324 | CHO | Cl | CN | 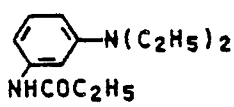 | blau |
| 325 | CN | Cl | CN | 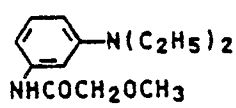 | blau |
| 326 | CN | Cl | CN | 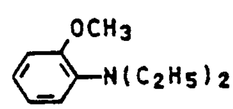 | blau |
| 327 | $NO_2$ | Cl | CN | 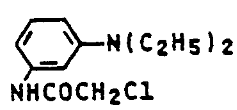 | grünst. blau |
| 328 | $NO_2$ | Cl | CN | 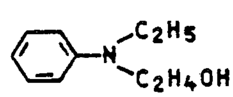 | blau |

| Bsp. Nr. | T | X | Y | -K | Farbe auf PES |
|---|---|---|---|---|---|
| 329 | $NO_2$ | Cl | CN | 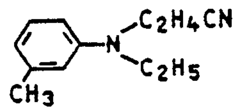 | blau |
| 330 | CN | Cl | CN | 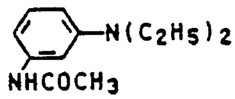 | blau |
| 331 | CN | Cl | CN | 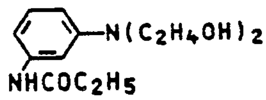 | blau |
| 332 | CN | Cl | CN | 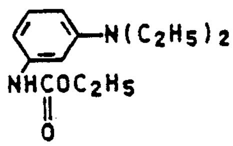 | blau |
| 333 | CHO | Cl | CN | 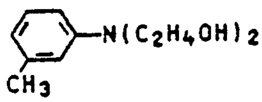 | blau |
| 334 | CHO | Cl | CN | 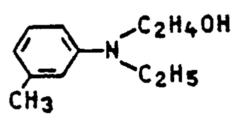 | blau |
| 335 | CHO | Cl | CN | 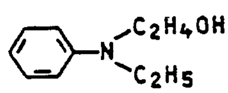 | rotst. blau |
| 336 | CHO | Cl | CN | 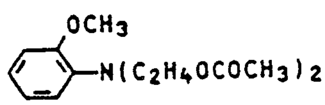 | blau |
| 337 | CHO | Cl | CN | 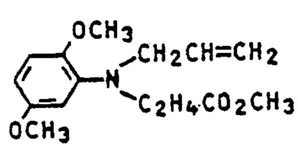 | blau |
| 338 | CHO | Br | CN | 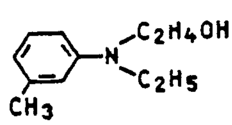 | blau |

| Bsp. Nr. | T | X | Y | -K | Farbe auf PES |
|---|---|---|---|---|---|
| 339 | CHO | Br | CN | 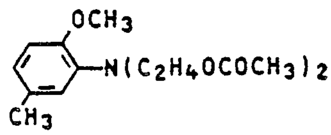 | blau |
| 340 | CHO | $C_6H_5S$ | CN | 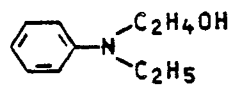 | rotst. blau |
| 341 | CHO | $C_6H_5S$ | CN | 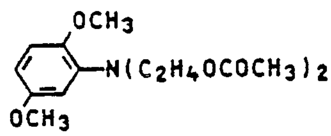 | grünst. blau |
| 342 | CHO | $OC_2H_5$ | CN | 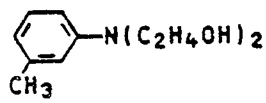 | rotst. blau |
| 343 | CHO | $C_6H_5SO_2$ | CN | 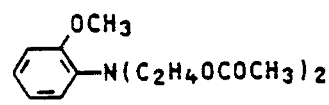 | blau |
| 344 | CHO | $C_6H_5SO_2$ | CN | 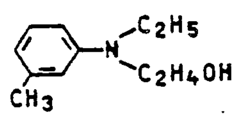 | blau |
| 345 | CN | Cl | CN | 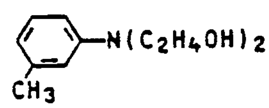 | rotst. blau |
| 346 | CN | Cl | CN | 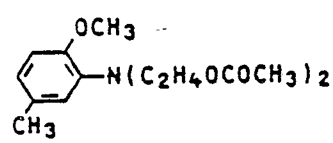 | blau |

| Bsp. Nr. | T | X | Y | -K | Farbe auf PES |
|---|---|---|---|---|---|
| 347 | $H_5C_2O_2C(CN)C{=}CH-$ | Cl | CN | 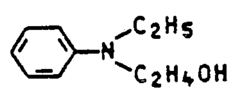 | blau |

Beispiel 348

2 Teile des in Beispiel 3 beschriebenen Farbstoffs wurden in 30 Vol.-Teilen DMF und 50 Vol.-Teilen Eisessig angerührt und mit 0,7 Teilen Anilin versetzt. Man rührte 12 Stunden bei Raumtemperatur und gab 500 Teile Wasser zu. Nach dem Absaugen erhielt man 2,2 Teile eines Pulvers der Formel

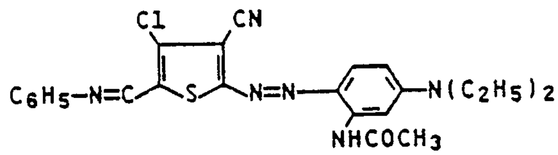

das Polyestergewebe in echten blauen Tönen färbt.

Beispiel 349

Zu 2,2 Teilen Phenylhydrazin in 40 Vol.-Teilen Ethanol wurden eine Suspension von 4 Teilen des in Beispiel 3 beschriebenen Farbstoffs in 50 Vol.-Teilen DMF und 2 Tropfen konz. Schwefelsäure gegeben. Nach 12 Stunden Rühren bei 25°C wurden 500 Teile Wasser zugesetzt und der Farbstoff der Formel

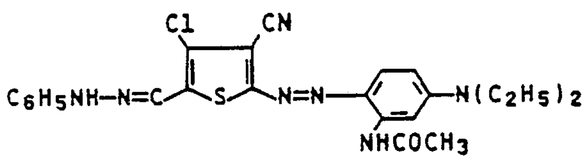

abgesaugt und getrocknet. Man erhielt 5 Teile schwarzes Pulver, das Polyestergewebe in echten, blauen Tönen färbt.

Beispiel 350

2,3 Teile des in Beispiel 23 beschriebenen Farbstoffs wurden mit 50 Vol.-Teilen Ameisensäure, 0,5 Teilen Hydroxylaminhydrochlorid und 0,5 Teilen Natriumformiat bei Raumtemperatur 12 Stunden gerührt. Nach Zugabe von 400 Teilen Wasser wurde abgesaugt. Man erhielt 2,4 Teile des Farbstoffs der Formel

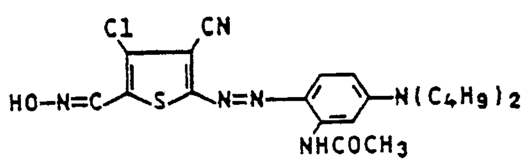 ,

der Polyestergewebe in echten blauen Tönen färbt.

Beispiel 351

18,7 Teile 2-Amino-3-cyan-4-chlor-5-formyl-thiophen werden in 120 Raumteilen Eisessig und 40 Raumteilen Propionsäure eine Stunde bei Raumtemperatur verrührt. Dann kühlt man auf 0/5°C ab, tropft dabei 31 Teile 42%ige Nitrosylschwefelsäure zu und rührt ungefähr eine Stunde bei 0/5°C nach.

Die eingesetzte Kupplungskomponente ist wie folgt erhalten worden: Ein Gemisch aus 17 Teilen wasserfeuchtem (ber. trocken) 2-Chlor-3-cyan-4-methyl-6-aminopyridin (erhalten z.B. nach dem in der DE—PS 2260827 beschriebenen Verfahren), 20 Volumenteilen Isobutanol, 18 Teilen 3-Aminopropyl-4-hydroxibutylether und 8 Teilen Soda wird am absteigenden Kühler unter guter Rührung 5 Stunden auf 145/150°C erhitzt, bis ein Dünnschichtchromatogramm vollständige Umsetzung anzeigt. Nach dem Abkühlen auf ungefähr 100°C wurden dann 35 Teile Essigsäure und bei 35/40°C unter weiterer leichter Kühlung 15 Teile 96%ige Schwefelsäure zugetropft. Nach dreistündigem Rühren waren dann ca. 92% der Hydroxiverbindung acetyliert. In die gut gerührte Mischung aus 100 Teilen der so erhaltenen Kupplungskomponenten, 300 Teilen Eis und 100 Teilen Wasser laßt man die Lösung des Diazoniumsalzes einlaufen und rührt ca. zwei Stunden bei 0/5°C nach, bis es verbraucht ist. Danach wird das Kupplungsgemisch abgesaugt, das Filtergut neutral gesachen und bei 80°C getrocknet. Das grünlich schwarze Pulver entspricht zu ca. 90% der Formel:

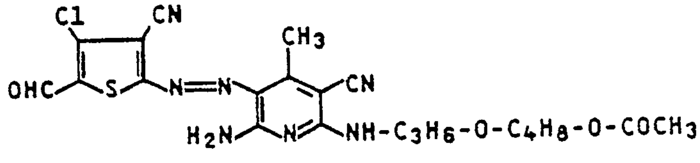

[$\lambda_{max}$: 546 nm (DMF/Eg. 9:1)] und enthält noch ca. 10% der nicht acetylierten Hydroxiverbindung.

Das Farbstoffgemisch besitzt sehr gute färberische Eigenschaften und er gibt auf Polyester sehr farbstarke, brillante, rotviolette Färbungen mit sehr hohen Licht- und Bügelechtheiten.

Beispiel 352

Wird unter den Herstellungsbedingungen des vorstehenden Beispiels die äquivalente Menge der nachstehend beschriebenen Kupplungskomponente verwendet, so erhält man den rotstichigen Blaufarbstoff ($\lambda_{max.}$: 572 nm — DMF/Eg. 9:1) der Formel:

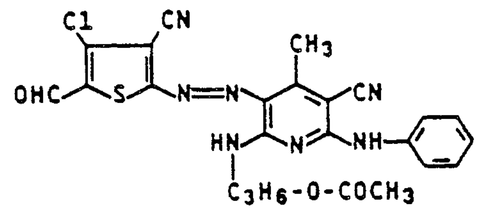

der noch ca. 5% der Hydroxiverbindung enthält.

Die verwendete Kupplungskomponente wurde wie folgt erhalten: 45 Teile 2-Chlor-3-cyan-4-methyl-6-(3'-hydroxi)-propylamino-pyridin (erhalten gemäß DP 2260827) werden in Gegenwart von einem Teil p-Toluolsulfosäure in 37 Teilen Anilin eine Stunde bei 125°C gerührt. Bei 135°C setzt man in 10minütigem Abstand in drei Portionen insgesamt 10 Teile Soda zu und rührt etwa 4 Stunden bei 145°C nach, bis ein DC vollständige Umsetzung anzeigt. Überschüssiges Anilin wird im Vakuum ausdestilliert und die heiße Schmelze auf 200 Raumteile heißes Wasser ausgetragen. Von der wäßrigen Lösung wird abdekantiert und die verbliebene Schmelze mehrfach mit heißem essigsauren Wasser verrührt und kalt abgetrennt. Nach dem Trocknen werden 14,2 Teile der Schmelze bestehend aus 2-Phenylamino-3-cyan-4-methyl-6-(3'-hydroxy)-propylamino-pyridin in 50 Raumteilen Eisessig aufgelöst und tropfenweise mit 6 Teilen 96%iger Schwefelsäure versetzt. Nach 3 stündigem Rühren bei 60°C wird die Lösung direkt in die beschriebene Kupplungsreaktion eingesetzt.

Analog wurden auch die in Tabelle I aufgeführten Farbstoffe erhalten, die ein vergleichbares Eigenschaftsprofil besitzen. Die $\lambda_{max}$-Werte wurden in einem Gemisch aus Dimethylformamid und Essigsäure 9:1 bestimmt.

Tabelle 1

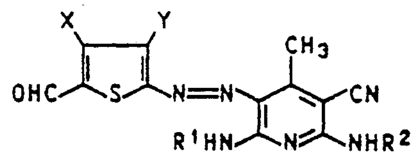

| Bsp. Nr. | X | Y | $R^1$ | $R^2$ | λmax [nm] |
|---|---|---|---|---|---|
| 353 | Cl | CN | H | $C_3H_6OC_4H_8OCOC_2H_5$ | 545,5 |
| 354 | Cl | CN | H | $C_3H_6OC_2H_7OC_2H_5$ | 547 |
| 355 | Cl | CN | H | $C_3H_6OC_2H_4OC_4H_9$ | 546,8 |
| 356 | Cl | CN | $C_2H_4OCH_3$ | 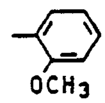 | 565 |
| 357 | Cl | CN | 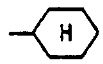 | 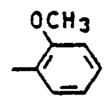 | 567 |
| 358 | Cl | CN | 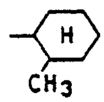 | 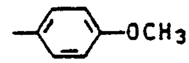 | 573 |
| 359 | $OC_2H_5$ | CN | H | $C_3H_6OC_4H_8OCOCH_3$ | 538 |
| 360 | $OC_2H_5$ | CN | H | $C_3H_6OC_2H_4OC_2H_5$ | 540 |
| 361 | $OC_2H_5$ | CN | $C_3H_6OCOCH_3$ | 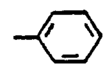 | 560,5 |
| 362 | $OC_2H_5$ | CN | $C_3H_6OH$ | 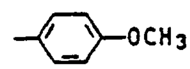 | 569 |
| 363 | 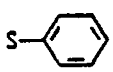 | CN | H | $C_3H_6OC_4H_8OCOCH_3$ | 540 |
| 364 | 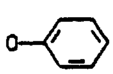 | CN | H | $C_3H_6OC_4H_8OCOCH_3$ | 540 |

Beispiel 365

14,6 Teile 2-Amino-3-cyan-4-ethoxi-5(2'-cyan-2'-carbethoxi)-vinyl-thiophen werden analog der Verfahrensweise des Beispiels 351, jedoch mit der Hälfte an Reagenzien und Hilfsmitteln diazotiert und auf 2-(4'-Acetoxi-butoxi)-3'-propylamino-3-cyan-4-methyl-6-amino-pyridin gekuppelt. Man erhält so ein blauschwarzes Pulver, das der Formel

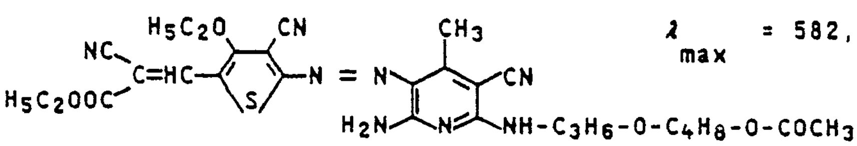

$\lambda_{max}$ = 582,3 nm (Aceton)

entspricht, aber noch ca. 10% der Hydroxiverbindung enthält und das Polyestermaterialien in mittelblauem Ton mit sehr guten Licht-, Wasch- und Bügelechtheiten färbt.

Die Diazokomponente wurde wie folgt erhalten: 60 Teile 2-Amino-3-cyan-4-ethoxi-thiophenaldehyd-5 wurden in 200 Raumteilen Dimethylformamid und 57 Teilen Cyanessigsäureethylester bei Raumtemperatur angerührt. Dazu tropfte man 10 Raumteile einer gesättigten wäßrigen Natriumacetat-Lösung und rührte 2 Stunden bei 40°C nach. Die Fällung wurde mit 1000 ml eiskaltem Methanol vervollständigt, abfiltriert und zunächst mit eiskaltem Methanol, dann mit Wasser gewaschen und bei 60°C getrocknet. Ausbeute: 60 Teile = 67.4%.

Beispiel 366

Nach der Verfahrensweise des Beispiels 351 jedoch mit der Hälfte der Reagenzien und Hilfsstoffe werden 14,1 Teile 2-Amino-3-cyan-4-chloro-5-(2′-cyan-2′-carbethoxi)-vinyl-thiophen diazotiert und auf 2-(4′-Acetoxibutoxi)-3′-propylamino-3-cyan-4-methyl-6-(3′-acetoxi)-propylamino-pyridin gekuppelt.

Man erhält so ein schwarzes Pulver, das zu ca. 90% der Formel

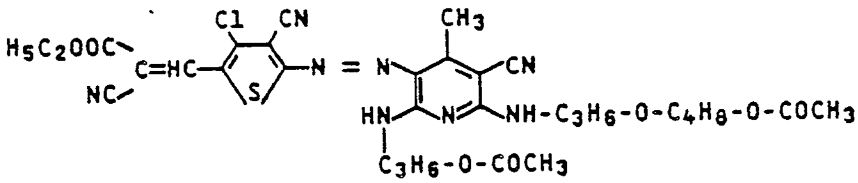

entspricht ($\lambda_{max}$ = 585,5 nm; Aceton) und Polyester in mittleren Blautönen mit sehr guten Licht-, Wasch- und Bügelechtheiten färbt.

Die Diazokomponente wurde in Analogie zu der des Beispiels 365 und die Kupplungskomponente wie folgt erhalten:

45 Teile 2-Chlor-3-cyan-4-methyl-6-(3′-hydroxi)-propylamino-pyridin (erhalten gemäß DP 2260827) wurden in Gegenwart von 14 Teilen Soda in 33 Teilen 3-Amino-propyl-4-hydroxi-butyl-ether fünf Stunden bei 145°C gerührt, auf 100°C gekühlt und auf 120 Raumteile Eisessig ausgetragen. Bei 30°C wurden dann 41 g 96%ige Schwefelsäure eigetropft. Nach 3stundigem Rühren bei 40°C wurde 1/4 der Lösung wie beschrieben zum Farbstoff umgesetzt.

Auf vergleichbare Weise wurden die Farbstoffe der Tabelle 2 mit ähnlich guten Eigenschaften erhalten. Die $\lambda_{max}$-Werte wurden in Aceton bestimmt, die mit * gekennzeichneten in einem Dimethylformamid/Essigsäure-Gemisch im Verhältnis 9:1.

Tabelle 2

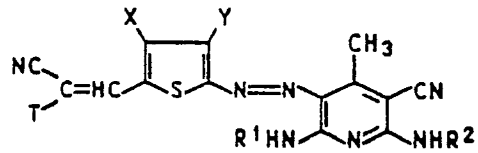

| Bsp. Nr. | T | X | Y | $R^1$ | $R^2$ | λmax [nm] |
|---|---|---|---|---|---|---|
| 367 | $COOC_2H_5$ | $OC_2H_5$ | CN | H | $C_3H_6OCH_3$ | 583 |
| 368 | $COOC_2H_5$ | $OC_2H_5$ | CN | H | $CH(CH_3)-CH_2-OCH_3$ | 580,5 |
| 369 | $COOC_2H_5$ | $OC_2H_5$ | CN | H | $C_3H_6OC_2H_4OC_2H_5$ | 594* |
| 370 | $COOC_2H_5$ | $OC_2H_5$ | CN | $C_3H_6OCH_3$ | $C_3H_6OC_4H_8OCOCH_3$ | 597* |
| 371 | $COOC_2H_5$ | $OC_2H_5$ | CN | $C_2H_4OH$ | $C_3H_6OC_2H_4OC_2H_5$ | 602,3* |
| 372 | $COOC_2H_5$ | $OC_2H_5$ | CN | $C_3H_6OC_2H_4OCH_3$ | $C_3H_4OCOCH_3$ | 591,5 |
| 373 | $COOC_2H_5$ | $OC_2H_5$ | CN | $C_2H_4OCOCH_3$ | $C_3H_6OC_2H_4OC_2H_5$ | 591,5 |
| 374 | $COOC_2H_5$ | $OC_2H_5$ | CN | $C_3H_6OCOCH_3$ | $C_3H_6OC_4H_8OCOCH_3$ | 594 |
| 375 | $COOC_2H_5$ | $OC_2H_5$ | CN | $C_3H_6OC_4H_8OCOCH_3$ | $C_3H_6OC_4H_8OCOCH_3$ | 594 |
| 376 | CN | $OC_2H_5$ | CN | H | $C_3H_6OC_4H_8OCOCH_3$ | 583 |
| 377 | CN | $OC_2H_5$ | CN | $C_3H_6OCH_3$ | $C_3H_6OC_4H_8OCOCH_3$ | 595 |
| 378 | CN | $OC_2H_5$ | CN | $C_3H_6OCOCH_3$ | $C_3H_6OC_4H_8OCOCH_3$ | 595 |
| 379 | CN | Cl | CN | $C_3H_6OCOCH_3$ | 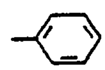 | 613,5* |
| 380 | CN | Cl | CN | $C_3H_4OH$ | $C_3H_6OC_2H_4OC_2H_5$ | 605,3* |
| 381 | CN | Cl | CN | H | $C_3H_6OC_2H_4OC_2H_5$ | 578 |
| 382 | $COOCH_3$ | Cl | CN | H | $C_3H_6OC_4H_8OCOCH_3$ | 577 |
| 383 | $COOC_2H_5$ | Cl | CN | H | $C_3H_6OCH_3$ | 578 |
| 384 | $COOC_2H_5$ | Cl | CN | H | $CH(CH_3)-CH_2-OCH_3$ | 575,5 |
| 385 | $COOC_2H_5$ | Cl | CN | H | 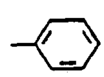 | 588 |
| 386 | CN | Cl | CN | $C_3H_6OCOCH_3$ | $C_3H_6OC_4H_8OCOCH_3$ | 586 |
| 387 | $COOC_2H_5$ | Cl | CN | $C_3H_6OCH_3$ | $C_3H_6OC_2H_4OC_4H_9$ | 588 |
| 388 | $COOC_2H_5$ | Cl | CN | $C_3H_6OCH_3$ | $C_3H_6OC_4H_8OCOCH_3$ | 585,5 |
| 389 | $COOC_2H_5$ | Cl | CN | $C_2H_4OCOCH_3$ | $C_3H_6OC_2H_4OC_2H_5$ | 586 |
| 390 | $COOC_2H_5$ | Cl | CN | $C_3H_6OC_4H_8OCOCH_3$ | 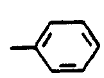 | 612* |
| 391 | CN | Cl | CN | $C_3H_6OC_4H_8OCOCH_3$ | $C_3H_6OC_4H_8OCOCH_3$ | 586 |

Beispiel 392

7,3 Teile 2-Amino-3-cyan-5-formyl-4-phenylsulfonyl-thiophen wurden bei macimal 30°C in 30 Vol.-Teilen 85%iger Schwefelsäure angerührt. Nach dem Zutropfen von 8,3 Teilen Nitrosylschwefelsäure (11,5% $N_2O_3$) bei 0—5°C wurde bei dieser Temperatur 4 Stunden gerührt.

Diese Diazoniumsalzlösung ließ man nach Verdünnen mit 20 ml konzentrierter Schwefelsäure bei 0°C in eine LÖsung von 6,6 Teilen 3-[N,N-Di-n-butyl]-amino-acetanilid in einer Mischung aus 25 Vol.-Teilen Dimethylformamid, 125 Teilen Wasser, 300 Teilen Eis und 0,5 Teilen Amidosulfonsäure langsam einfließen. Nach Beendigung der Kupplung wurde der Farbstoff abgesaugt, neutral gewaschen und getrocknet. Man erhielt 12 Teile des Farbstoffs der Formel

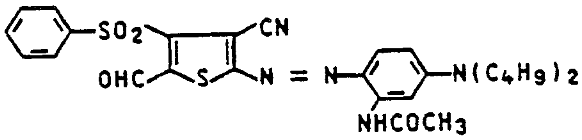

der Polyesterfasern in blauen, echten Nuancen färbt.

Beispiel 393

7,3 Teile 2-Amino-3-cyan-5-formyl-4-phenylsulfonyl-thiophen wurden analog dem vorstehenden Beispiel diazotiert.

Die Diazoniumsalzlösung wurde bei 0°C in eine Lösung von 5,2 Teilen 3-(N,N-Diethyl)-amino-acetanilid in einer Mischung aus 125 Teilen Waser 350 Teilen Eis, 10 Vol.-Teilen 32%iger Salzsäure und 0,5 Teilen Amidosulfonsäure eigetropft. Nach Beendigung der Kupplung wurde der Farbstoff abgesaugt, neutral gewaschen und getrocknet. Man erhielt 10,5 Teile eines Pulvers der Formel

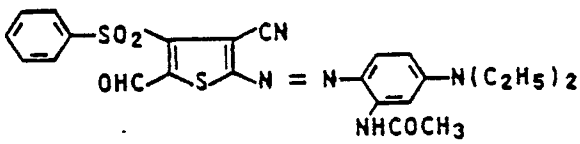

das Polyestergewebe in echten, blauen Tönen färbt.

Analog erhält man die in der folgenden Tabelle gekennzeichneten Farbstoffe.

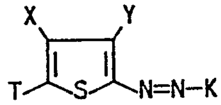

| Bsp. Nr. | T | X | Y | −K | Färbung auf Polyester |
|---|---|---|---|---|---|
| 394 | CHO | $C_6H_5SO_2$ | CN | 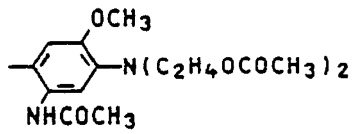 | türkis |
| 395 | CHO | $C_6H_5SO_2$ | CN | 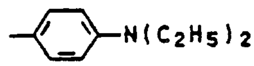 | blauviolett |
| 396 | CHO | $C_6H_5SO_2$ | CN | 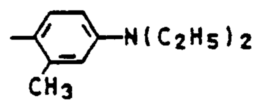 | blau |
| 397 | CHO | $C_6H_5SO_2$ | CN | 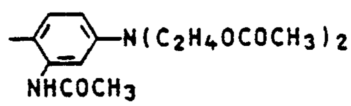 | blau |
| 398 | CHO | $C_6H_5SO_2$ | CN | 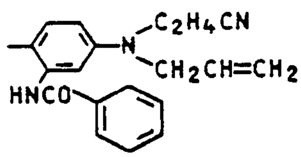 | blau |
| 399 | CHO | $C_6H_5SO_2$ | CN | 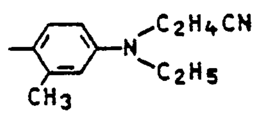 | rotstichig blau |
| 400 | CHO | $C_6H_5SO_2$ | CN | 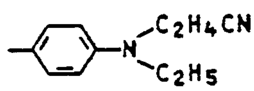 | blauviolett |
| 401 | CHO | $C_6H_5SO_2$ | CN | 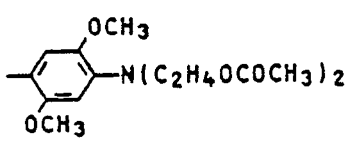 | grünstichig blau |

| Bsp. Nr. | T | X | Y | −K | Färbung auf Polyester |
|---|---|---|---|---|---|
| 402 | CHO | $C_6H_5SO_2$ | CN | 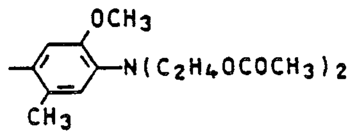 | blau |
| 403 | CHO | $C_6H_5SO_2$ | CN | 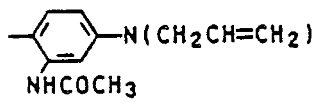 | blau |
| 404 | CHO | $C_6H_5SO_2$ | $CO_2C_2H_5$ | 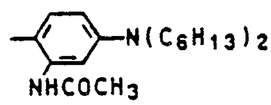 | blau |
| 405 | CHO | $C_6H_5SO_2$ | CN | 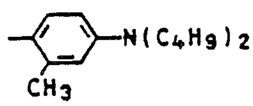 | blau |
| 406 | CHO. | $C_6H_5SO_2$ | CN | 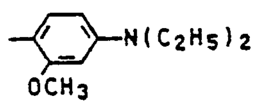 | blau |
| 407 | CHO | $C_6H_5SO_2$ | CN | 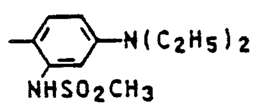 | blau |
| 408 | CHO | $C_6H_5SO_2$ | CN | 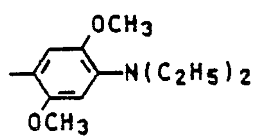 | blau |
| 409 | CHO | $C_6H_5SO_2$ | CN | 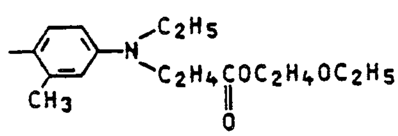 | blau |
| 410 | CHO | $C_6H_5SO_2$ | CN | 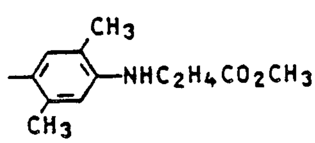 | blau |

| Bsp. Nr. | T | X | Y | -K | Färbung auf Polyester |
|---|---|---|---|---|---|
| 411 | CHO | 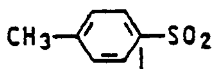 | $CO_2C_2H_5$ | 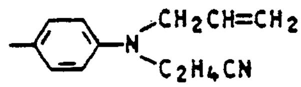 | rotstichig blau |
| 412 | CHO | $C_6H_5SO_2$ | $CO_2C_2H_5$ | 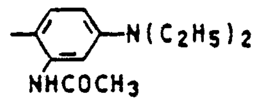 | blau |
| 413 | CHO | $C_6H_5SO_2$ | $CONH_2$ | 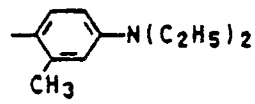 | blau |
| 414 | CHO | $C_6H_5SO_2$ | $NO_2$ | 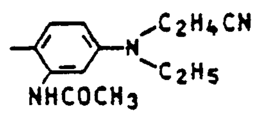 | blau |
| 415 | CHO | $C_6H_5SO_2$ | $CON(CH_3)_2$ | 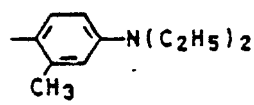 | blau |
| 416 | CHO | $C_6H_5SO_2$ | $SO_2CH_3$ | 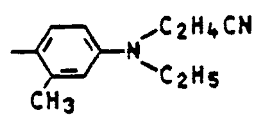 | blau |
| 417 | CHO | $CH_3SO_2$ | $CO_2C_2H_5$ | 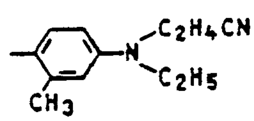 | blau |
| 418 | CHO | $C_6H_5SO_2$ | CN | 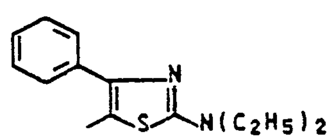 | blau |
| 419 | CHO | $C_6H_5SO_2$ | CN | 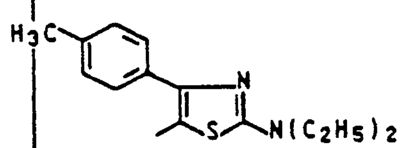 | blau |
| 420 | CHO | $CH_3SO_2$ | CN | 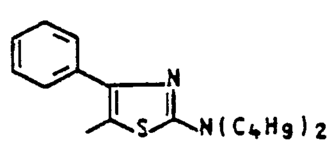 | blau |

| Bsp. Nr. | T | X | Y | -K | Färbung auf Polyester |
|---|---|---|---|---|---|
| 421 | CHO | $C_6H_5SO_2$ | CN | 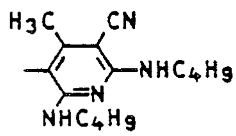 | violett |
| 422 | CHO | $C_6H_5SO_2$ | CN | 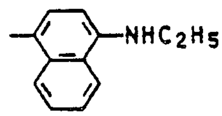 | blau |
| 423 | CHO | $C_6H_5SO_2$ | $CO_2C_2H_5$ | 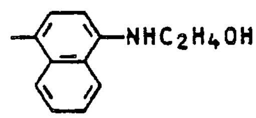 | blau |
| 424 | CHO | $C_6H_5SO_2$ | CN | 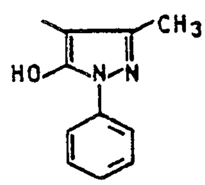 | gelbbraun |
| 425 | CHO | $C_6H_5SO_2$ | CN | 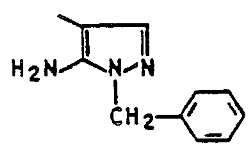 | rot |
| 426 | CHO | $C_6H_5SO_2$ | CN | 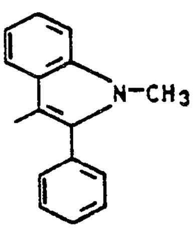 | rot |
| 427 | CHO | $C_6H_5SO_2$ | CN | 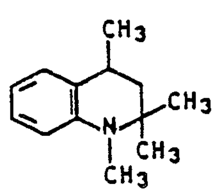 | blau |
| 428 | CHO | $C_6H_5SO_2$ | CN | 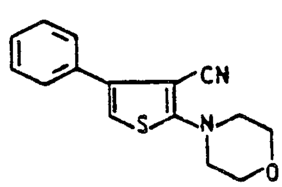 | blau |

| Bsp. Nr. | T | X | Y | −K | Färbung auf Polyester |
|---|---|---|---|---|---|
| 429 | CHO | p−Cl−$C_6H_4SO_2$ | CN | 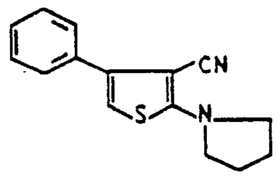 | blau |
| 430 | CHO | $C_6H_5SO_2$ | CN | 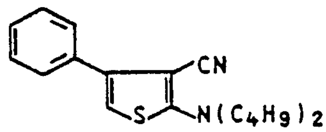 | blau |
| 431 | CHO | $C_6H_5SO_2$ | CN | 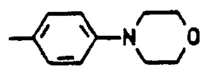 | violett |
| 432 | CHO | $C_6H_5SO_2$ | CN | 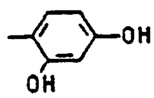 | rot |
| 433 | CHO | $C_6H_5SO_2$ | CN | 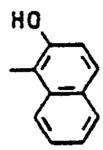 | rot |
| 434 | CN | $C_6H_5SO_2$ | CN | 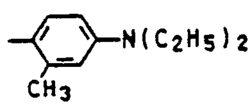 | blau |
| 435 | CN | $C_6H_5SO_2$ | CN | 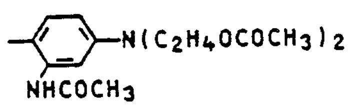 | blau |
| 436 | CN | $C_6H_5SO_2$ | CN | 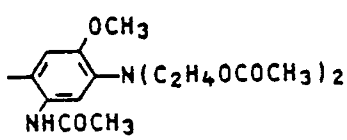 | türkis |
| 437 | CN | $CH_3SO_2$ | CN | 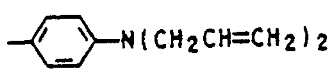 | rotstichig blau |
| 438 | CN | $C_6H_5SO_2$ | CN | 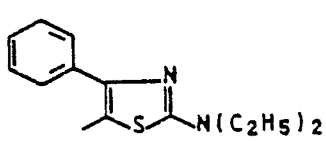 | blau |
| 439 | $NO_2$ | $C_6H_5SO_2$ | CN | 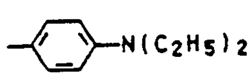 | rotstichig blau |

| Bsp. Nr. | T | X | Y | –K | Färbung auf Polyester |
|---|---|---|---|---|---|
| 440 | $NO_2$ | $C_6H_5SO_2$ | CN | 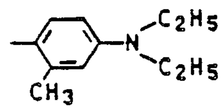 | blau |
| 441 | $NO_2$ | $C_6H_5SO_2$ | CN | 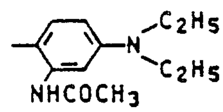 | blau |
| 442 | $NO_2$ | $C_6H_5SO_2$ | CN | 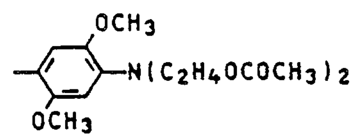 | grünstichig blau |
| 443 | $CH_3\underset{\underset{O}{\|}}{C}-$ | $C_6H_5SO_2$ | CN | 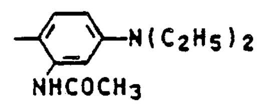 | blau |

Beispiel 444

Zu 2,3-Teilen 4-Chlor-3-cyan-5-formyl-2-(4'-N,N-dibutylamino-2'-acetylamino-phenylazo)-thiophen in 50 Volumenteilen Dimethylformamid wurden 1,2 Teile Natrium-Phenylsulfinat zugegeben und bei Raumtemperatur gerührt. Nach beendeter Umsetzung (Dünnschichtchromatogramm) wurde die Reaktionsmischung auf 300 Teile Wasser gegeben und der entstandene Farbstoff isoliert. Man erhielt 2,4 Teile des Farbstoffs der Formel

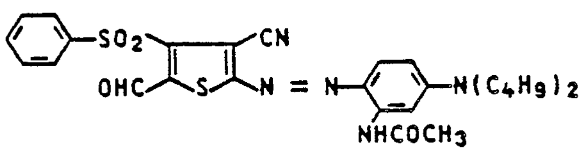

der Polyesterfasern in blauen, echten Nuancen färbt.

Analog Beispiel 444 wurden die in der folgenden Tabelle angegebenen Farbstoffe erhalten.

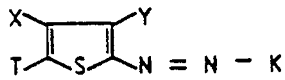

| Bsp. Nr. | T | X | Y | −K | Färbung auf Polyester |
|---|---|---|---|---|---|
| 445 | CHO | $C_6H_5SO_2$ | CN | 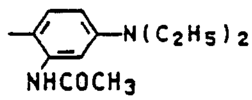 | blau |
| 446 | CHO | $C_6H_5SO_2$ | CN | 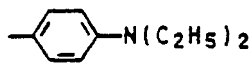 | blauviolett |
| 447 | CHO | $C_6H_5SO_2$ | CN | 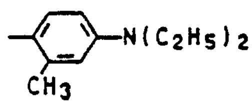 | blau |
| 448 | CHO | $C_6H_5SO_2$ | CN | 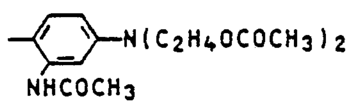 | blau |
| 449 | CHO | $C_6H_5SO_2$ | CN | 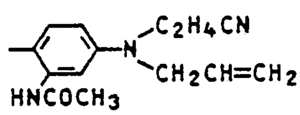 | blau |
| 450 | CHO | $C_6H_5SO_2$ | CN | 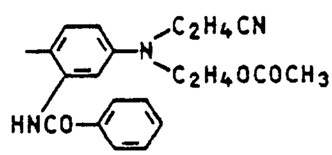 | blau |
| 451 | CHO | $C_6H_5SO_2$ | CN | 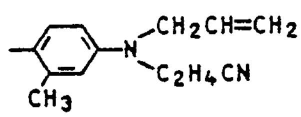 | rotstichig blau |
| 452 | CHO | $C_6H_5SO_2$ | CN | 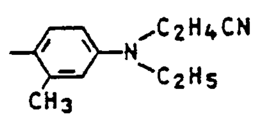 | rotstichig blau |
| 453 | CHO | $C_6H_5SO_2$ | $CO_2C_2H_5$ | 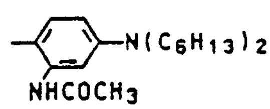 | blau |

Beispiel 454

2,6-Teile des in Beispiel 405 beschriebenen Farbstoffes wurden in 80 Vol.-Teilen Dioxan gelöst, mit 1,1 Teilen Cyanessigsäureethylester, 0,4 Teilen Eisessig und 0,4 Teilen Piperidin versetzt und 16 Stunden bei Raumtemperatur gerührt. Anschließend wurden 50 Teile Wasser und 50 Teile Eis zugegeben. Es wurde 15 Minuten gerührt, das Produkt abgesaugt und neutral gewaschen. Nach dem Trocknen im Vakuum bei 50°C erhielt man 2,8 Teile des Farbstoffs der Formel

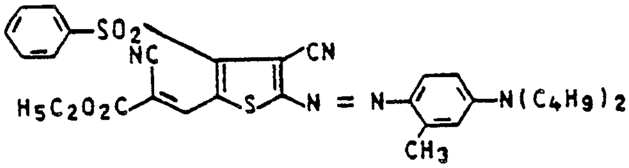

der Polyesterfasern in echten, blauen Tönen färbt.

Beispiel 455

2,3 Teile des in Beispiel 395 beschriebenen Farbstoffs wurden in 70 Vol.-Teilen Dioxan gelöst, mit 1,4 Teilen Cyanessigsäurebutylester, 0,4 Teilen Eisessig und 0,4 Teilen Piperidin versetzt und 16 Stunden bei Raumtemperatur gerührt. Anschließend wurden 50 Teile Wasser und 50 Teile Eis zugegeben, 1 Stunde gerührt, das Produkt abgesaugt und neutral gewaschen. Man erhielt nach dem Trocknen 2,5 Teile des Farbstoffs der Formel

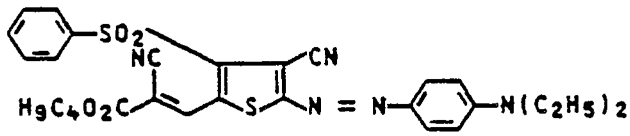

der Polyestergewebe in echten, blauen Tönen färbt.

Analog den obigen Beispielen wurden die in der folgenden Tabelle aufgeführten Farbstoffe erhalten.

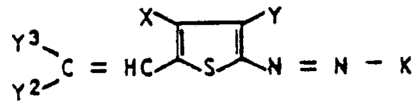

| Bsp Nr. | X | Y | $Y^2$ | $Y^3$ | −K | Färbung auf PES |
|---|---|---|---|---|---|---|
| 456 | $C_6H_5SO_2$ | CN | CN | CN | 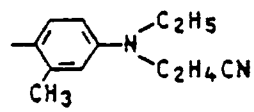 | blau |
| 457 | $C_6H_5SO_2$ | CN | $COCH_3$ | $COOC_2H_5$ | 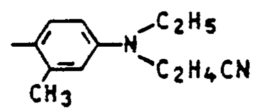 | blau |
| 458 | $C_6H_5SO_2$ | $COOC_2H_5$ | CN | $COOC_2H_5$ | 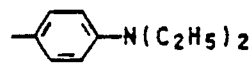 | blau |
| 459 | $C_6H_5SO_2$ | $COOC_2H_5$ | CN | $COOC_2H_5$ | 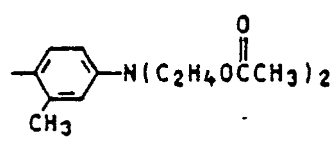 | blau |
| 460 | $C_6H_5SO_2$ | $COOC_2H_5$ | CN | $COOC_2H_5$ | 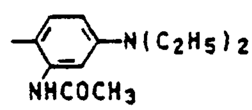 | blau |
| 461 | $C_6H_5SO_2$ | CN | CN | $COOC_2H_5$ | 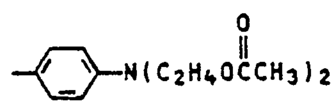 | rot-stichig blau |
| 462 | $C_6H_5SO_2$ | CN | CN | $COOC_2H_5$ | 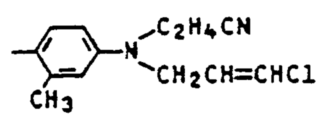 | blau |
| 463 | $C_6H_5SO_2$ | CN | CN | $COOC_2H_5$ | 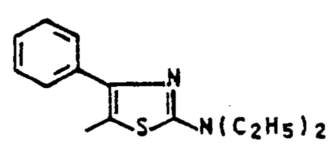 | blau |
| 464 | $C_6H_5SO_2$ | CN | CN | $COOC_2H_5$ | 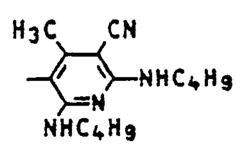 | blau |
| 465 | $C_6H_5SO_2$ | CN | CN | $COOC_2H_5$ | 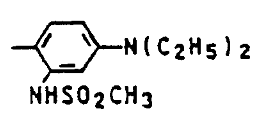 | blau |

| Bsp Nr. | X | Y | $Y^2$ | $Y^3$ | –K | Färbung auf PES |
|---|---|---|---|---|---|---|
| 466 | $C_6H_5SO_2$ | CN | CN | $COOC_2H_5$ | 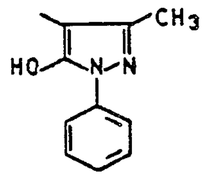 | violett |
| 467 | $C_6H_5SO_2$ | CN | CN | $COOC_2H_5$ | 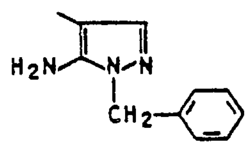 | violett |
| 468 | $C_6H_5SO_2$ | CN | CN | $COOC_2H_5$ | 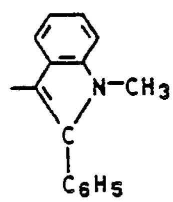 | violett |
| 469 | $C_6H_5SO_2$ | CN | CN | $CONHCH_3$ | 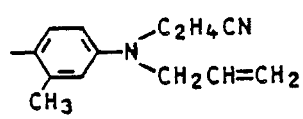 | blau |
| 470 | $C_6H_5SO_2$ | CN | CN | $CONHC_2H_5$ | 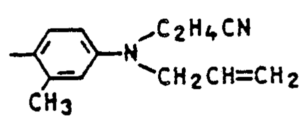 | blau |
| 471 | $C_6H_5SO_2$ | CN | CN | $SO_2CH_3$ | 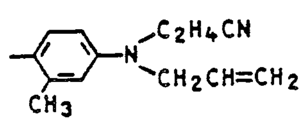 | blau |
| 472 | $C_6H_5SO_2$ | CN | CN | $COCH_3$ | 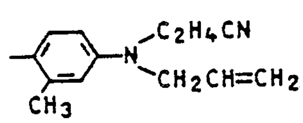 | blau |
| 473 | $C_6H_5SO_2$ | CN | CN | $COOCH_3$ | 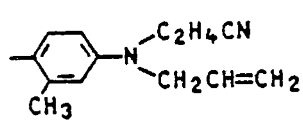 | blau |
| 474 | $C_6H_5SO_2$ | CN | CN | $COOC_4H_9(n)$ | 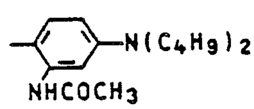 | türkis |
| 475 | $C_6H_5SO_2$ | CN | CN | $COOC_4H_9(n)$ | 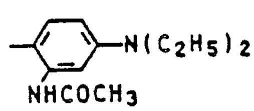 | grünstichig blau |

| Bsp Nr. | X | Y | $Y^2$ | $Y^3$ | -K | Färbung auf PES |
|---|---|---|---|---|---|---|
| 476 | $C_6H_5SO_2$ | CN | CN | $COOC_4H_9(n)$ | 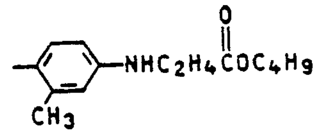 | blau |
| 477 | $C_6H_5SO_2$ | CN | CN | $COOC_4H_9(n)$ | 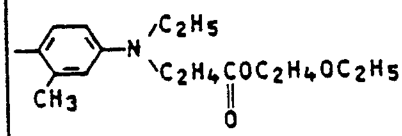 | blau |
| 478 | $C_6H_5SO_2$ | CN | CN | 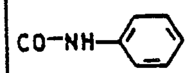 | 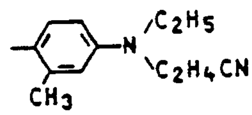 | blau |
| 479 | $C_6H_5SO_2$ | CN | CN | 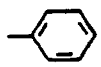 | 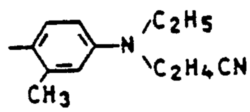 | blau |
| 480 | $C_6H_5SO_2$ | CN | CN | 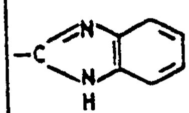 | 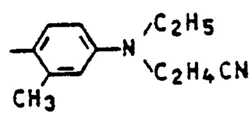 | blau |
| 481 | $C_6H_5SO_2$ | CN | CN | $CONH_2$ | 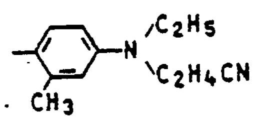 | blau |
| 482 | $CH_3SO_2$ | CN | CN | $COOC_2H_5$ | 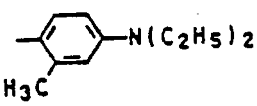 | blau |
| 483 | $CH_3SO_2$ | CN | CN | $COOC_2H_5$ | 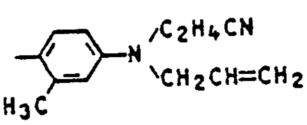 | blau |
| 484 | $CH_3SO_2$ | CN | CN | $COOC_2H_5$ | 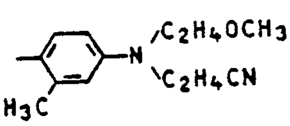 | blau |
| 485 | $p-Cl-C_6H_4SO_2$ | CN | CN | 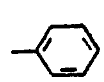 | 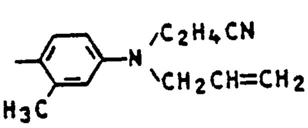 | blau |

Beispiel 486

a) 14,5 Teile 2-Amino-3-cyan-5-formyl-4-phenylsulfonyl-thiophen wurden in 50 Vol.-Teilen Ethanol suspendiert und mit 1 Teil Eisessig sowie 1 Teil Pioeridin versetzt. Anschließend ließ man bei Raumtemperatur 13 Vol.-Teile Cyanessigsäureethylester zutropfen und rührte 5 Stunden bei 60°C. Die

Reaktionsmischung wurde auf 800 Teile Wasser gegeben, der Niederschlag abgesaugt, mit Wasser gewaschen und bei 50°C im Vakuum getrocknet. Man erhielt 16 Teile 2-Amino-3-cyan-4-phenylsulfonyl-5-(β-cyan-β-carboethoxy)-vinyl-thiophen der Formel

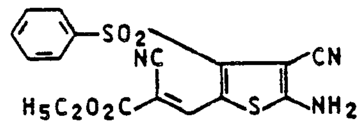

das ohne weitere Reinigung umgesetzt wurde.

b) 3,9 Teile 2-Amino-3-cyan-4-phenylsulfonyl-5-(β-cyan-β-carboethoxy)-vinyl-thiophen wurden in 35 Vol.-Teilen Eisessig/Propionsäure (3:1) angerührt und bei 0—5°C langsam mit 3,5 Teilen Nitrosylschwefelsäure (11,5% $N_2O_3$) versetzt. Nach 2 h bei 0—5°C ließ man die Diazoniumsalzlösung in eine Lösung von 2 Teilen N-Cyanethyl-N-ethyl-m-toluidin in einer Mischung aus 25 Teilen Wasser, 100 Teilen Eis, 5 Vol.-Teilen 32%iger Salzsäure und 0,2 Teilen Amidosulfonsäure einfließen. Nach Beendigung der Kupplung wurde der Farbstoff abgesaugt neutral gewaschen und im Vakuum getrocknet. Man erhielt 4,5 Teile des Farbstoffs der Formel

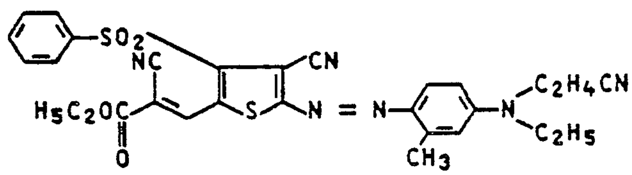

der auf Polyesterfasern blaue Färbungen mit allgemein sehr guten Echtheiten ergiht.

Beispiel 487

3,4 Teile 2-Amino-3-cyan-4-phenylsulfonyl-5-(β,β-dicyan)-vinyl-thiophen wurden in 25 Vol.-Teilen Phosphorsäure (85%) suspendiert, bei 0—5°C langsam mit 3,5 Teilen Nitrosylschwefelsäure (11,5% $N_2O_3$) umgesetzt und 2 h bei 0—5°C gerührt. Diese Diazoniumsalzlösung ließ man in eine Mischung aus 1,5 Teilen N,N-Diethylanilin, 20 Teilen Wasser, 60 Teilen Eis, 5 Vol.-Teilen 32%iger Salzsäure und 0,2 Teile Amidosulfonsäure langsam einfließen. Nach beendeter Kupplung wurde die Suspension filtriert, der Feststoff neutral gewaschen und getrocknet. Man erhielt 4,2 Teile des Farbstoffs der Formel

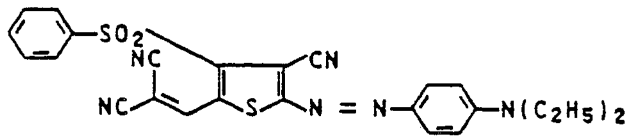

der auf Polyester blaue Färbungen mit allgemein guten Echtheiten ergibt.

Analog wurden auch die in der nachfolgenden Tabelle gekennzeichneten Verbindungen erhalten.

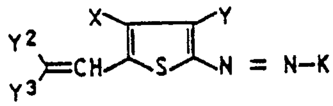

| Bsp. Nr. | X | Y | $Y^2$ | $Y^3$ | -K | Färbung auf PES |
|---|---|---|---|---|---|---|
| 488 | $CH_3SO_2$ | CN | CN | $COOCH_3$ | 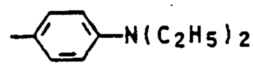 | blau |
| 489 | $C_6H_5SO_2$ | CN | CN | $COOCH_3$ | 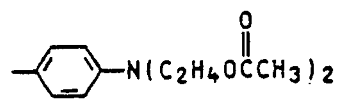 | blau |
| 490 | $C_6H_5SO_2$ | CN | CN | $COOCH_3$ | 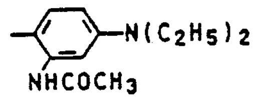 | grünstichig blau |
| 491 | $C_6H_5SO_2$ | CN | CN | $COOCH_3$ | 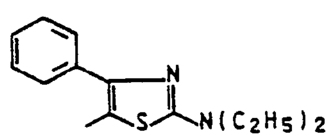 | türkis |
| 492 | $C_6H_5SO_2$ | CN | CN | $COOC_2H_5$ | 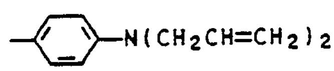 | blau |
| 493 | $C_6H_5SO_2$ | CN | CN | $COOC_2H_5$ | 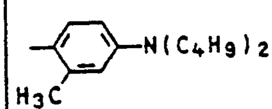 | blau |
| 494 | $C_6H_5SO_2$ | CN | CN | $COOC_2H_5$ | 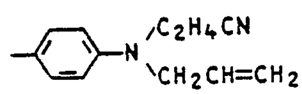 | blau |
| 495 | $C_6H_5SO_2$ | CN | CN | $COOC_2H_5$ | 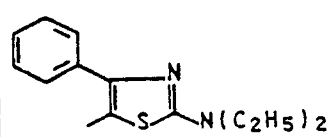 | türkis |
| 496 | $C_6H_5SO_2$ | CN | CN | $CONHCH_3$ | 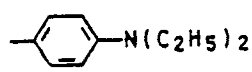 | blau |
| 497 | $C_6H_5SO_2$ | $COOCH_3$ | CN | $COOC_2H_5$ | 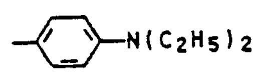 | blau |

Als ätzbare Farbstoffe kommen z.B. die Verbindungen der Beispiele 21, 22, 105 und 106 sowie die der Beispiele 333—347 in Betracht.

**Patentansprüche**

1. Verbindungen der allgemeinen Formel I

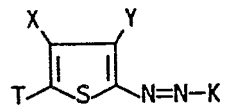

in der

X Fluor, Chlor, Brom, $SO_2E$, OH, $OCH_3$, $OC_2H_5$, $OC_3H_7$, $OC_4H_9$, $OCH_2C_6H_5$, $OC_6H_{11}$, $OC_6H_5$, $OC_6H_4CH_3$, $OC_6H_4Cl$, SH, $SCH_3$, $SC_2H_5$, $SC_3H_7$, $SC_4H_9$, $SCH_2C_6H_5$, $SC_2H_4OH$, $SCH_2COOCH_3$, $SCH_2COOC_2H_5$, $SC_6H_{11}$, $SC_6H_5$ oder $SC_6H_4CH_3$,

E Alkyl, Alkenyl, Cycloalkyl, Aralkyl, Aryl, Chlor, OH, $CH_3O$, $C_2H_5O$, $C_3H_7O$, $C_4H_9O$, $C_6H_5—CH_2O$, $C_6H_5—CH_2—CH_2O$, $C_6H_5O$, $ClC_6H_4O$, $CH_3C_6H_4O$, $NH_2$, $NHCH_3$, $N(CH_3)_2$, $NHC_2H_5$, $N(C_2H_5)_2$, $NHC_4H_9$, $N(C_4H_9)_2$, $NHC_6H_5$, $NHC_6H_4—CH_3$, $NHC_6H_4Cl$ oder $NCH_3C_6H_5$.

Y Cyan, Nitro, Alkanoyl, Aroyl, Alkylsulfonyl, Arylsulfonyl, Carboxyl, Carbonester oder gegebenenfalls substituiertes Carbamoyl,

T Wasserstoff, $C_1$- bis $C_4$-Alkyl, NO, $NO_2$, $SO_3H$, CHO, CN, $CH_3CO$, $C_2H_5CO$, $C_6H_5CO$, $CH_3SO_2$, $C_2H_5SO_2$, $C_6H_5SO_2$ oder ein Rest der Formel —CH=B, wobei B der Rest einer methylenaktiven Verbindung oder eines Amins ist, und

K den Rest einer Kupplungskomponenten bedeutet.

2. Verbindungen gemäß Anspruch 1 bei denen K ein Rest der allgemeinen Formeln

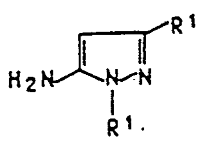 , 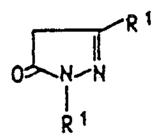 , 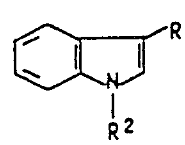 , 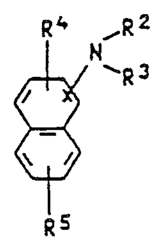 , 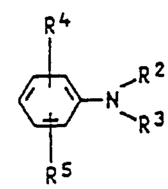 ,

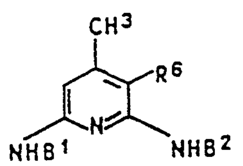 , 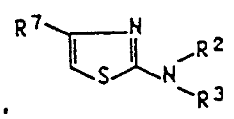 , 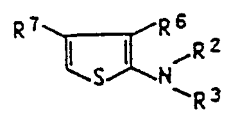 oder 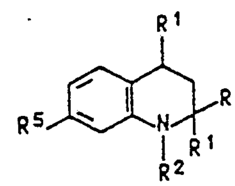 ,

ist in denen

$B^1$ Wasserstoff oder $B^2$

$B^2$ gegebenenfalls substituiertes Alkyl, Cycloalkyl, Alkenyl, Aryl oder Acyl,

$R^1$ Wasserstoff, Alkyl, Aralkyl oder Aryl,

$R^2$ Wasserstoff oder $R^3$,

$R^3$ gegebenenfalls substituiertes Alkyl, Cycloalkyl, Alkenyl, Aralkyl oder Aryl,

$R^4$ und $R^5$ unabhängig voneinander Wasserstoff, Alkyl, Alkoxy, Phenoxy, Halogen, Alkylsulonylamino, Dialkylaminosulfonylamino oder Acylamino,

$R^6$ Cyan, Carbamoyl, Nitro, Acetyl oder Carbalkoxy und

$R^7$ gegebenenfalls substituiertes Phenyl, Alkyl, Aralkyl, Halogen, $C_1$- bis $C_{10}$-Alkoxy, Phenoxy, Benzyloxy, $C_1$- bis $C_4$-Alkoxy-ethoxy, $C_1$- bis $C_4$-Alkyl- oder Phenylmercapto sind.

3. Verbindungen gemäß Anspruch 1 der Formeln Ia

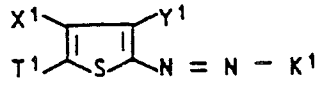 und I b 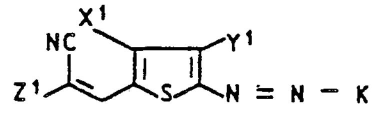

in denen

$X^1$ Chlor, Brom, Hydroxy, $C_1$- bis $C_4$-Alkoxy oder -Alkylthio, Methylsulfonyl, Phenylsulfonyl, Phenoxy oder Phenylthio,

$Y^1$ Cyan, Carbonester oder substituiertes Carbamoyl,

$T^1$ Formyl, Nitro, Cyan,

$Z^1$ Cyan, Carbonester oder substituiertes Carbamoyl und

$K^1$ der Rest einer Kupplungskomponente der Anilin-, Thiazol-, Pyrazol-, Thiophen- oder Pyridinreihe sind.

4. Verbindungen gemäß Anspruch 1 der Formel

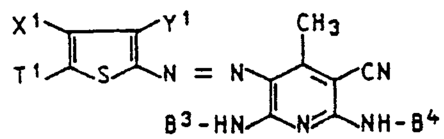

in der

$X^1$, $Y^1$ und $T^1$ die für Anspruch 3 angegebene Bedeutung haben und

$B^3$ Wasserstoff oder $B^4$ und

$B^4$ gegebenenfalls durch Sauerstoff unterbrochenes und gegebenenfalls durch Hydroxyl, $C_1$- bis $C_4$-Alkanoyloxi, $C_1$- bis $C_4$-Alkoxi, Benzyloxi oder Phenoxi substituiertes $C_2$- bis $C_8$-Alkyl oder gegebenenfalls durch Methyl oder Methoxi substituiertes Phenyl sind.

5. Verbindungen gemäß Anspruch 1 der Formel

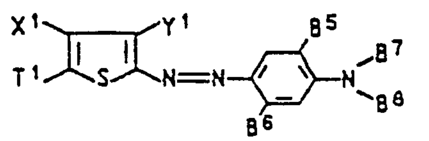

in der

$B^5$ Wasserstoff, Chlor, Methyl, Methoxy oder Ethoxy,

$B^6$ Wasserstoff, Methyl, Methoxy oder $C_1$- bis $C_4$-Alkanoylamino, das noch durch Methoxy, Ethoxy, Phenoxy, Chlor oder Cyan substituiert sein kann,

$B^7$ Wasserstoff oder $B^8$ und

$B^8$ gegebenenfalls durch Hydroxy, $C_1$- bis $C_4$-Alkoxy, $C_1$- bis $C_4$-Alkoxycarbonyl, $C_1$- bis $C_4$-Alkanoyloxy oder Cyan substituiertes $C_1$- bis $C_6$-Alkyl, Cyclohexyl, Allyl, Benzyl oder Phenyl sind und $X^1$, $Y^1$ und $T^1$ die für Anspruch 3 angegebene Bedeutung haben.

6. Verbindungen gemäß Anspruch 1, bei denen Y Cyan ist.

7. Verbindungen gemäß Anspruch 1, wobei X Chlor, Methoxy, Ethoxy, Phenylthio, Methylsulfonyl oder Phenylsulfonyl und T Formyl sind.

8. Verbindungen gemäß Anspruch 1, wobei X Chlor, Methoxy, Ethoxy, Phenylthio, Methylsulfonyl oder Phenylsulfonyl und T Cyan sind.

9. Verbindungen gemäß Anspruch 1, wobei X Chlor, Methoxy, Ethoxy, Phenylthio, Methylsulfonyl oder Phenylsulfonyl und T Nitro sind.

10. Verbindungen gemäß Anspruch 1, wobei X Chlor, Methoxy, Ethoxy, Phenylthio, Methylsulfonyl oder Phenylsulfonyl und T ein Rest der Formel CH=B ist, wobei B

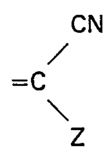

bedeutet und Z Wasserstoff, Cyan, Carboxyl, Carbonester, gegebenenfalls substituiertes Carbamoyl, Benzimidazolyl, Benzoxazolyl oder Benzthiazolyl ist.

11. Verwendung der Verbindungen gemäß Anspruch 1 zum Färben synthetischer Fasern oder von Kunststoffen.

**Revendications**

1. Composés de la formule générale I

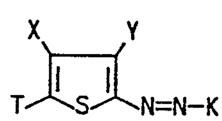

dans laquelle

X représente un atome de fluor, de chlore, de brome, un radical $SO_2E$, OH, $OCH_3$, $OC_2H_5$, $OC_3H_7$, $OC_4H_9$, $OCH_2C_6H_5$, $OC_6H_{11}$, $OC_6H_5$, $OC_6H_4CH_3$, $OC_6H_4Cl$, SH, $SCH_3$, $SC_2H_5$, $SC_3H_7$, $SC_4H_9$, $SCH_2C_6H_5$, $SC_2H_4OH$,

$SCH_2COOCH_3$, $SCH_2COOC_2H_5$, $SC_6H_{11}$, $SC_6H_5$ ou $SC_6H_4CH_3$,

E représente un radical alkyle, alcényle, cycloalkyle, aralkyle, aryle, un atome de chlore, un groupe OH, $CH_3O$, $C_2H_5O$, $C_3H_7O$, $C_4H_9O$, $C_6H_5—CH_2O$, $C_6H_5—CH_2—CH_2O$, $C_6H_5O$, $ClC_6H_4O$, $CH_3C_6H_4O$, $NH_2$, $NHCH_3$, $N(CH_3)_2$, $NHC_2H_5$, $N(C_2H_5)_2$, $NHC_4H_9$, $N(C_4H_9)_2$, $NHC_6H_5$, $NHC_6H_4—CH_3$, $NHC_6H_4Cl$ ou $NCH_3C_6H_5$,

Y représente un radical cyano, nitro, alcanoyle, aroyle, alkylsulfonyle, arylsulfonyle, carboxyle, ester carboxylique, ou carbamoyle éventuellement substitué,

T représente un atome d'hydrogène, un radical alkyle en $C_1$ à $C_4$, NO, $NO_2$, $SO_3H$, CHO, CN, $CH_3CO$, $C_2H_5CO$, $C_6H_5CO$, $CH_3SO_2$, $C_2H_5SO_2$, $C_6H_5SO_2$, ou un reste de la formule —CH=B dans laquelle B représente le reste d'un composé à fonction méthylène active, ou d'une amine, et

K représente le reste d'un composant de copulation.

2. Composés suivant la revendication 1, dans lesquels K représente un reste des formules générales

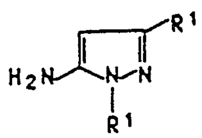 , 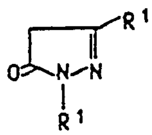 , 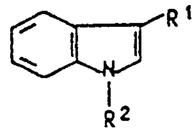 , 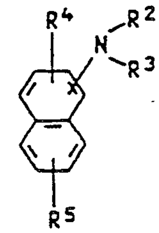 , 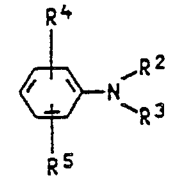 ,

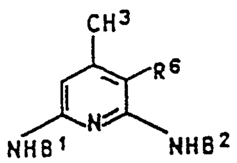 , 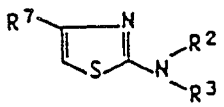 , 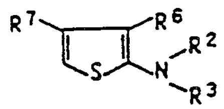 ou 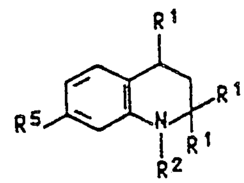 ,

dans lesquelles

$B^1$ représente un atome d'hydrogène ou $B^2$,

$B^2$ représente un radical acyle, aryle, alcényle, cycloalkyle, alkyle, éventuellement substitué,

$R^1$ représente un atome d'hydrogène, un radical alkyle, aralkyle ou aryle,

$R^2$ représente un atome d'hydrogène ou $R^3$, $R^3$ représente un radical aryle, aralkyle, alcényle, cycloalkyle ou alkyle, éventuellement substitué,

$R^4$ et $R^5$ représentent, chacun indépendamment l'un de l'autre, un atome d'hydrogène, un radical alkyle, alcoxy, phénoxy, un atome d'halogène, un radical alkylsulfonylamino, dialkylaminosulfonylamino ou acylamino,

$R^6$ représente un radical cyano, carbamoyle, nitro, acétyle ou carbalcoxy et

$R^7$ représente un atome d'halogène, un radical alralkyle, alkyle, phényle éventuellement substitué, un groupe alcoxy en $C_1—C_{10}$, phénoxy, benzyloxy, alcoxyéthoxy en $C_1—C_4$, alkyl($C_1—C_4$)- ou phénylmercapto.

3. Composés selon la revendication 1, des formules Ia

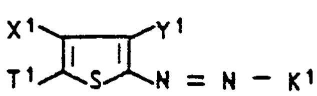 et I b 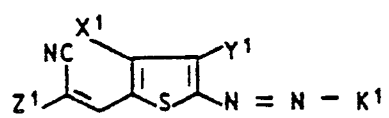

dans lesquelles

$X^1$ représente un atome de chlore, un atome de brome, un radical hydroxyle, alcoxy en $C_1—C_4$ ou alkylthio en $C_1—C_4$, méthylsulfonyle, phénylsulfonyle, phénoxy ou phénylthio,

$Y^1$ représente un radical cyano, ester carboxylique ou carbamoyle substitué,

$T^1$ représente un radical formyle, nitro, cyano,

$Z^1$ représente un radical cyano, ester carboxylique ou carbamoyle substitué et

$K^1$ représente le reste d'un composant de copulation de la série de l'aniline, du thiazole, du pyrazole, du thiophène ou de la pyridine.

4. Composés suivant la revendication 1 de la formule

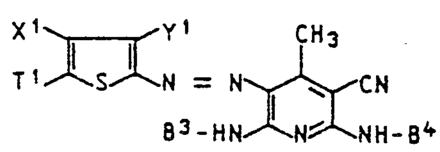

dans laquelle

$X^1$, $Y^1$ et $T^1$ possèdent les significations qui leur ont été attribuées à propos de la définition de la

revendication 3 et

$B^3$ représente un atome d'hydrogène ou $B^4$ et

$B^4$ représente un radical alkyle en $C_2$ à $C_8$, éventuellement interrompu par de l'oxygène et éventuellement à substitution hydroxylique, alcanoyloxylique en $C_1$—$C_4$, alcoxylique en $C_1$—$C_4$, benzyloxylique ou phénoxylique, ou un radical phényle à substitution méthylique ou méthoxylique éventuelle.

5. Composés suivant la revendication 1 de la formule

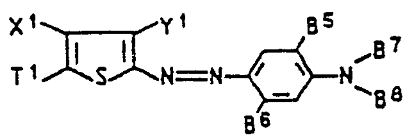

dans laquelle

$B^5$ représente un atome d'hydrogène, de chlore, le radical méthyle, méthoxy ou éthoxy,

$B^6$ représente un atome d'hydrogène, un radical méthyle, méthoxy ou alcanoylamino en $C_1$—$C_4$, éventuellement méthoxy-, éthoxy-, phénoxy-, chloro- ou cyano-substitué,

$B^7$ représente un atome d'hydrogène ou $B^8$ et

$B^8$ représente un radical phényle, benzyle, allyle, cyclohexyle ou alkyle en $C_1$—$C_6$, éventuellement hydroxy-, alcoxy($C_1$—$C_4$)-, alcoxy($C_1$—$C_4$)carbonyl-, alcanoyl($C_1$—$C_4$)oxy- ou cyano-substitué $X^1$, $Y^1$ et $T^1$ possèdent les significations qui leur ont été attributées à propos de la définition de la revendication 3.

6. Composés suivant la revendication 1, dans lesquels Y représente le radical cyano.

7. Composés suivant la revendication 1, dans lesquels X représente un atome de chlore, un radical méthoxy, éthoxy, phénylthio, méthylsulfonyle ou phénylsulfonyle et T représente le radical formyle.

8. Composés suivant la revendication 1, dans laquelle X représente un atome de chlore, un radical méthoxy, éthoxy, phénylthio, méthylsulfonyle ou phénylsulfonyle et T représente le radical cyano.

9. Composés suivant la revendication 1, dans lesquels X représente un atome de chlore, un radical méthoxy, éthoxy, phénylthio, méthylsulfonyle ou phénylsulfonyle et T représente le radical nitro.

10. Composés suivant la revendication 1, dans lesquels X représente un atome de chlore, un radical méthoxy, éthoxy, phénylthio, méthylsulfonyle ou phénylsulfonyle et T représente un reste de la formule CH=B, dans laquelle B représente un radical

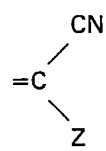

et Z représente un atome d'hydrogène, un radical cyano, carboxyle, ester carboxylique, carbamoyle éventuellement substitué, benzimidazolyle, benzoxazolyle ou benzothiazolyle.

11. Utilisation des composés selon la revendication 1, pour la teinture de matières plastiques ou de fibres synthétiques.

**Claims**

1. A compound of the formula I

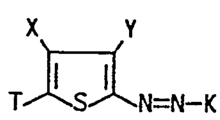

where X is fluorine, chlorine, bromine, $SO_2E$, OH, $OCH_3$, $OC_2H_5$, $OC_3H_7$, $OC_4H_9$, $OCH_2C_6H_5$, $OC_6H_{11}$, $OC_6H_5$, $OC_6H_4CH_3$, $OC_6H_4Cl$, SH, $SCH_3$, $SC_2H_5$, $SC_3H_7$, $SC_4H_9$, $SCH_2C_6H_5$, $SC_2H_4OH$, $SCH_2COOCH_3$, $SCH_2COOC_2H_5$, $SC_6H_{11}$, $SC_6H_5$ or $SC_6H_4CH_2$, E is alkyl, alkenyl, cycloalkyl, aralkyl, aryl, chlorine, OH, $CH_3O$, $C_2H_5O$, $C_3H_7O$, $C_4H_9O$, $C_6H_5$—$CH_2O$, $C_6H_5$—$CH_2$—$CH_2O$, $C_6H_5O$, $ClC_6H_4O$, $CH_3C_6H_4O$, $NH_2$, $NHCH_3$, $N(CH_3)_2$, $NHC_2H_5$, $N(C_2H_5)_2$, $NCH_4H_9$, $N(C_4H_9)_2$, $NHC_6H_5$, $NHC_6H_4$—$CH_3$, $NHC_6H_4Cl$ or $NCH_3C_6H_5$, Y is cyano, nitro, alkanoyl, aroyl, alkylsulfonyl, arylsulfonyl, carboxyl, a carboxylic ester group or unsubstituted or substituted carbamoyl, T is hydrogen, $C_1$—$C_4$-alkyl, NO, $NO_2$, $SO_3H$, CHO, CN, $CH_3CO$, $C_2H_5CO$, $C_6H_5CO$, $CH_3SO_2$, $C_2H_5SO_2$, $C_6H_5SO_2$ or a radical of the formula —CH=B, in which B is a radical of a methylene active compound or of an amine, and K is a radical or a coupling component.

2. A compound as claimed in claim 1, in which K is a radical of the formulae

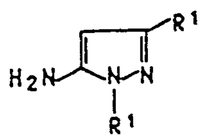 , 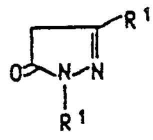 , 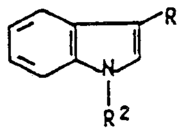 , 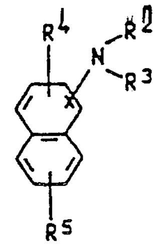 , 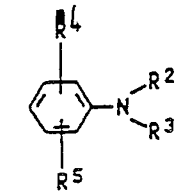 ,

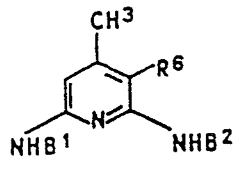 , 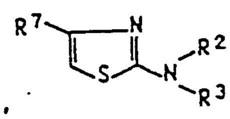 , 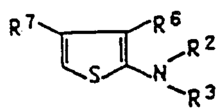 or 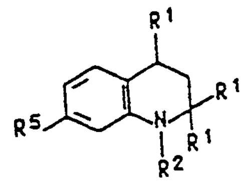 ,

where $B^1$ is hydrogen or $B^2$, $B^2$ is unsubstituted or substituted alkyl, cycloalkyl, alkenyl, aryl or acyl, $R^1$ is hydrogen, alkyl, aralkyl, or aryl, $R^2$ is hydrogen or $R^3$, $R^3$ is unsubstituted or substituted alkyl, cycloalkyl, alkenyl, aralkyl or aryl, $R^4$ and $R^5$ independently of one another are each hydrogen, alkyl, alkoxy, phenoxy, halogen, alkylsulfonylamino, dialkylaminosulfonylamino or acylamino, $R^6$ is cyano, carbamoyl, nitro, acetyl or carbalkoxy and $R^7$ is unsubstituted or substituted phenyl, alkyl, aralkyl, halogen, $C_1$—$C_{10}$-alkoxy, phenoxy, benzyloxy, $C_1$—$C_4$-alkoxyethoxy, $C_1$—$C_4$-alkylmercapto or phenylmercapto.

3. A compound as claimed in claim 1, of the formula Ia

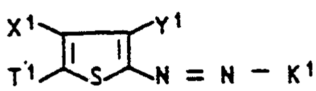 and I b 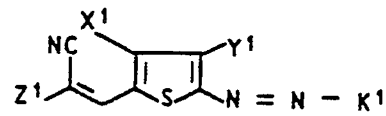

where $X^1$ is chlorine, bromine, hydroxyl, $C_1$—$C_4$-alkoxy or -alkylthio, methylsulfonyl, phenylsulfonyl, phenoxy or phenylthio, $Y^1$ and $Z^1$ are each cyano, a carboxylic ester group or substituted carbamoyl, $T^1$ is formyl, nitro or cyano and $K^1$ is a radical of a coupling component of the aniline, thiazole, pyrazole, thiophene or pyridine series.

4. A compound as claimed in claim 1, of the formula

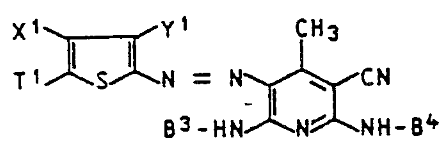

where $X^1$, $Y^1$ and $T^1$ have the meanings stated for claim 3, $B^3$ is hydrogen or $B^4$ and $B^4$ is $C_2$—$C_8$-alkyl which may or may not be interrupted by oxygen and is unsubstituted or substituted by hydroxyl, $C_1$—$C_4$-alkanoyloxy, $C_1$—$C_4$-alkoxy, benzyloxy or phenoxy or is phenyl which is unsubstituted or substituted by methyl or methoxy.

5. A compound as claimed in claim 1, of the formula

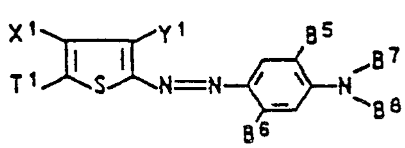

where $B^5$ is hydrogen, chlorine, methyl, methoxy, or ethoxy, $B^6$ is hydrogen, methyl, methoxy, or $C_1$—$C_4$-alkanoylamino which may be further substituted by methoxy, ethoxy, phenoxy, chlorine, or cyano, $B^7$ is hydrogen or $B^8$ and $B^8$ is cyclohexyl, allyl, benzyl, phenyl or $C_1$—$C_6$-alkyl which is unsubstituted or substituted by hydroxyl, $C_1$—$C_4$-alkoxy, $C_1$—$C_4$-alkoxycarbonyl, $C_1$—$C_4$-alkanoyloxy or cyano, and $X^1$, $Y^1$ and $T^1$ have the meanings stated for claim 3.

6. A compound as claimed in claim 1, wherein Y is cyano.

7. A compound as claimed in claim 1, wherein X is chlorine, methoxy, ethoxy, phenylthio, methylsulfonyl or phenylsulfonyl and T is formyl.

8. A compound as claimed in claim 1, wherein X is chlorine, methoxy, ethoxy, phenylthio, methylsulfonyl or phenylsulfonyl and T is cyano.

9. A compound as claimed in claim 1, wherein X is chlorine, methoxy, ethoxy, phenylthio, methylsulfonyl or phenylsulfonyl and T is nitro.

10. A compound as claimed in claim 1, wherein X is chlorine, methoxy, ethoxy, phenylthio, methylsulfonyl or phenylsulfonyl and T is a radical of the formula CH=B, in which B is

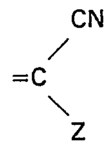

and Z is hydrogen, cyano, carboxyl, a carboxylic ester group, unsubstituted or substituted carbamoyl, benzimidazolyl, benzoxazolyl or benzothiazolyl.

11. The use of the compound as claimed in claim 1 for dyeing synthetic fibers or plastics.